# FASCICULE DE BREVET EUROPEEN

(11) **EP 1 077 927 B1**
(45) Date de publication et mention de la délivrance du brevet: **07.12.2005**
(21) Numéro de dépôt: 99918036.7
(22) Date de dépôt: 10.05.1999
(51) Int. Cl.: C07C 233/36, C07C 233/40, C07C 317/32, C07C 323/40, C07C 323/41, C07C 323/44, C07D 209/60, C07D 221/10, C07D 295/12, C07D 307/81, C07D 333/58, C07D 335/08, C07D 407/12, C07D 471/04, C07D 495/04, A61K 31/165

(54) **NOUVEAUX COMPOSES CYCLIQUES SUBSTITUES, LEUR PROCEDE DE PREPARATION ET LES COMPOSITIONS PHARMACEUTIQUES QUI LES CONTIENNENT**
SUBSTITUIERTE CYCLISCHE VERBINDUNGEN, VERFAHREN ZU IHRER HERSTELLUNG SOWIE SIE ENTHALTENDE PHARMAZEUTISCHE ZUSAMMENSETZUNGEN
NOVEL SUBSTITUTED CYCLIC COMPOUNDS, PREPARATION METHOD AND PHARMACEUTICAL COMPOSITIONS CONTAINING THEM

(30) Priorité: 12.05.1998 FR 9805957
(43) Date de publication de la demande: 28.02.2001
(73) Titulaire: Les Laboratoires Servier, 92200 Neuilly sur Seine (FR)
(72) Inventeur: LESIEUR, Daniel, F-59147 Gondecourt (FR); KLUPSCH, Frédérique, F-62640 Montigny en Gohelle (FR); GUILLAUMET, Gérald, F-45650 Saint Jean le Blanc (FR); VIAUD, Marie-Claude, F-45000 Orléans (FR); LANGLOIS, Michel, F-92330 Sceaux (FR); BENNEJEAN, Caroline, F-94220 Charenton Le Pont (FR); RENARD, Pierre, F-78150 Le Chesnay (FR); DELAGRANGE, Philippe, F-92130 Issy les Moulineaux (FR)
(86) Numéro de dépôt international: PCT/FR1999/001100
(87) Numéro de publication internationale: WO 1999/058495

(56) Documents cités:
- EP-A- 0 530 087
- EP-A- 0 562 956
- EP-A- 0 662 471
- EP-A- 0 728 738
- EP-A- 0 745 583
- EP-A- 0 745 584

## Description

La présente invention concerne de nouveaux dérivés cycliques substitués, leur procédé de préparation et les compositions pharmaceutiques qui les contiennent.

On connaît, dans l'art antérieur, des amides indoliques thiosubstituées utiles en tant qu'agents anti-inflammatoires (EP 624575, EP 535923), en tant qu'antagonistes de la libération de gonadotropine (WO 9721703), en tant qu'antagonistes 5HT-2B ou 2C (WO 9602537), ou en tant qu'intermédiaires de synthèse (Akad. Nauk Gruz., 1991, 141 (3), pp. 545-8 ; Pept. Chem., 1993, 31, pp. 33-6, J. Pharm. Sci., 1973, 62 (8), pp. 1374-5).

Des composés benzo[b]thiophéniques ont également été décrits en tant qu'agents anti-inflammatoires (US 5350748, US 5068248) ou anticancéreux (Heterocycles, 1985, 23 (5), pp. 1173-80).

Les composés de la présente invention sont nouveaux et présentent des caractéristiques pharmacologiques très intéressantes concernant les récepteurs mélatoninergiques.

De nombreuses études ont mis en évidence ces dix dernières années le rôle capital de la mélatonine (N-acétyl-5-méthoxytryptamine) dans de nombreux phénomènes physiopathologiques ainsi que dans le contrôle du rythme circadien. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.
Outre leur action bénéfique sur les troubles du rythme circadien (J. Neurosurg. 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp 264-272) et analgésiques (Pharmacopsychiat., 1987, 20, pp 222-223) ainsi que pour le traitement des maladies de Parkinson (J. Neurosurg. 1985, 63, pp 321-341) et d'Alzheimer (Brain Research, 1990, 528, pp 170-174). De même, ces composés ont montré une activité sur certains cancers (Melatonin - Clinical Perspectives, Oxford University Press, 1988, pp 164-165), sur l'ovulation (Science 1987, 227, pp 714-720), sur le diabète (Clinical Endocrinology, 1986, 24, pp 359-364), et dans le traitement de l'obésité (International Journal of Eating Disorders, 1996, 20 (4), pp 443-446).
Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol. Sci., 1995, 16, p 50 ; WO 97.04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands spécifiques. De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Les composés de la présente invention outre leur nouveauté, montrent une très forte affinité pour les récepteurs de la mélatonine et/ou une sélectivité pour l'un ou l'autre des sous-types réceptoriels mélatoninergiques.

La présente invention concerne plus particulièrement les composés de formule (I) :

R-A-R' (I) (I)

dans laquelle :
◆ A représente :
   ― un système cyclique de formule (II) : 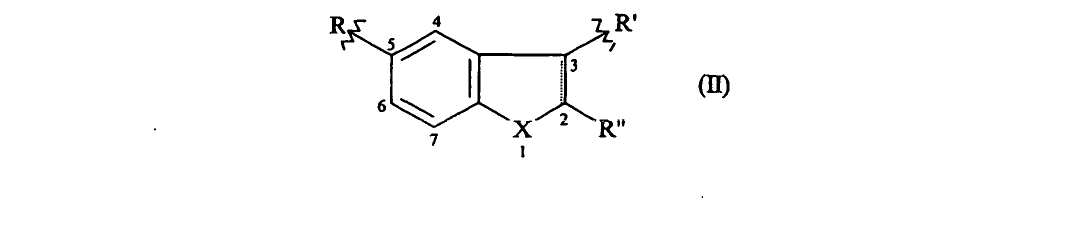 où
      - X représente un atome d'oxygène ou de soufre, ou un groupement NR₀ (dans lequel R₀ représente un atome d'hydrogène, un groupement alkyle (C₁-C₆) linéaire ou ramifié, aryle, arylalkyle (C₁-C₆) linéaire ou ramifié ou SO₂Ph),
      - la représentation .... signifie que la liaison peut être simple ou double étant entendu que la valence des atomes est respectée,
      - R" représente un groupement Rₐ, ORₐ, CORₐ, COORₐ, OCORₐ, OSO₂CF₃, cyano, nitro ou atomes d'halogène,
      où Rₐ représente un atome d'hydrogène, un groupement alkyle (C₁-C₆) linéaire ou ramifié substitué ou non, alkényle (C₂-C₆) linéaire ou ramifié substitué ou non, alkynyle (C₂-C₆) linéaire ou ramifié substitué ou non, polyhalogénoalkyle (C₁-C₆) linéaire ou ramifié, cycloalkyle (C₃-C₈) substitué ou non, cycloalkyle (C₃-C₈) alkyle (C₁-C₆) linéaire ou ramifié substitué ou non, cycloalkényle (C₃-C₈) substitué ou non, cycloalkényle (C₃-C₈) alkyle (C₁-C₆) linéaire ou ramifié substitué ou non, aryle, arylalkyle (C₁-C₆) linéaire ou ramifié, arylalkényle (C₁-C₆) linéaire ou ramifié, hétéroaryle, hétéroarylalkyle (C₁-C₆) linéaire ou ramifié, hétéroarylalkényle (C₁-C₆) linéaire ou ramifié, hétérocycloalkyle substitué ou non, hétérocycloalkényle substitué
      ou non, hétérocycloalkylalkyle (C₁-C₆) linéaire ou ramifié substitué ou non, ou hétérocycloalkényl alkyle (C₁-C₆) linéaire ou ramifié substitué ou non,
   ― ou un système cyclique de formule (III) : 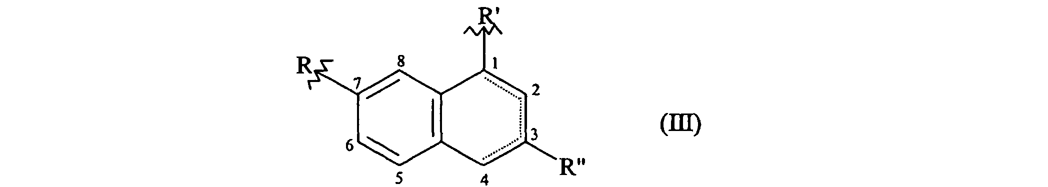 où R" et la représentation .... ont la même signification que précédemment,
◆ R représente :
   un groupement -NR'ₐR"ₐ dans lequel R'ₐ et R"ₐ, identiques ou différents, peuvent prendre toutes les valeurs de Rₐ et peuvent également former, avec l'atome d'azote qui les porte un groupement cyclique contenant 5 à 10 chaînons pouvant comporter, en plus de l'atome d'azote, un à trois hétéroatomes choisis parmi oxygène, soufre et azote,
   ◆ et R' représente un groupement de formule (VII) :

      ―G―R² (VII)

      où
      - G représente une chaîne alkylène -(CH₂)ₜ- (dans laquelle t vaut 2 ou 3),
      - et R² représente un groupement 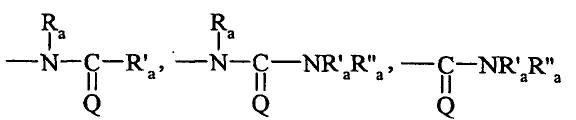
      ou 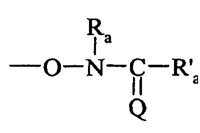 dans lesquels Q représente un atome d'oxygène ou de soufre, et Rₐ, R'ₐ et R"ₐ (identiques ou différents) sont définis de la même façon que précédemment, R'ₐ et R"ₐ pouvant former avec l'atome d'azote qui les porte un groupement cyclique tel que défini précédemment,
   étant entendu que :
      - par "hétérocycloalkyle" on entend tout groupement saturé mono ou polycyclique contenant de 5 à 10 atomes contenant 1 à 3 hétéroatomes choisis parmi azote, oxygène ou soufre,
      - par "hétérocycloalkényle" on entend tout groupement mono ou polycyclique non aromatique contenant une ou plusieurs insaturations, contenant de 5 à 10 atomes et pouvant contenir 1 à 3 hétéroatomes choisis parmi azote, oxygène ou soufre,
      - le terme "substitué" affecté aux expressions "alkyle", "alkényle", "alkynyle", signifie que ces groupements sont substitués par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, amino ou atomes d'halogène,
      - le terme "substitué" affecté aux expressions "cycloalkyle", "cycloalkylalkyle", "cycloalkényle", "cycloalkénylalkyle", "hétérocycloalkyle", "hétérocycloalkényle", "hétérocycloalkylalkyle", "hétérocycloalkénylalkyle", signifie que la partie cyclique de ces groupements est substituée par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, amino ou atomes d'halogène,
      - par "aryle" on entend tout groupement aromatique mono ou polycyclique contenant 6 à 22 atomes de carbone, ainsi que le groupement biphényle,
      - par "hétéroaryle" on entend tout groupement aromatique mono ou polycyclique contenant de 5 à 10 atomes contenant 1 à 3 hétéroatomes choisis parmi azote, oxygène ou soufre, les groupements "aryle" et "hétéroaryle" pouvant être substitués par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié,
      cyano, nitro, amino ou atomes d'halogène,
   étant entendu que :
      - lorsque A représente un noyau indole substitué en position 2 par un phényle éventuellement substitué, alors R² ne peut représenter un groupement -NHCOR_{c} dans lequel R_{c} est un groupement contenant un hétérocycle mono ou bicyclique aromatique
      ou non,
leurs énantiomères et diastéréoisomères, ainsi que leurs sels d'addition à un acide ou une base pharmaceutiquement acceptable.

Parmi les acides pharmaceutiquement acceptables, on peut citer à titre non limitatif les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique, etc...

Parmi les bases pharmaceutiquement acceptables, on peut citer à titre non limitatif l'hydroxyde de sodium, l'hydroxyde de potassium, la triéthylamine, la tertbutylamine, etc...

De façon avantageuse, l'invention concerne les composés pour lesquels A représente un système cyclique de formule (II) comme par exemple benzothiophène, dihydrobenzothiophène, benzofurane, dihydrobenzofurane, indole ou indoline, ou de formule (III) comme par exemple naphtalène ou tétrahydronaphtalène.

De façon encore plus avantageuse, les substituants R préférés de l'invention sont ceux représentés par un groupement NR'ₐR"ₐ où R'ₐ et R"ₐ (identiques ou différents) représentent un atome d'hydrogène, un groupement alkyle, polyhalogénoalkyle, alkényle, alkynyle, cycloalkyle, aryle, arylalkyle, hétéroaryle ou hétéroarylalkyle, comme par exemple méthyle, éthyle, propyle, isopropyle, butyle, isobutyle, secbutyle, tertbutyle, pentyle, isopentyle, hexyle, trifluorométhyle, vinyle, allyle, propargyle, phényle, naphtyle, benzyle, phénéthyle, cyclopropyle, cyclobutyle, cyclopentyle, cyclohexyle, cyclohexényle, méthylcyclopropyle, éthylcyclopropyle, furyle, thiényle, pyridyle, furylméthyle, pyridylméthyle, ou forment avec l'atome d'azote qui les porte un groupement piperazine; piperidine, morpholine ou thiomorpholine.

Les substituants R' préférés de l'invention sont ceux pour lesquels G représente une chaîne alkylène -(CH₂)ₜ- où t vaut 2 ou 3, et R² représente un groupement 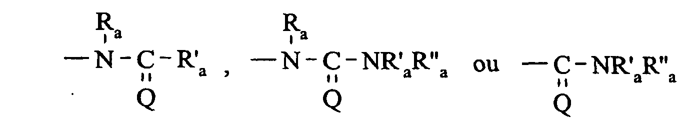 dans lesquels Q, Rₐ, R'ₐ et R"ₐ sont tels que définis précédemment.

De façon encore plus avantageuse, les substituants R' préférés de l'invention sont ceux pour lesquels G représente un groupement -(CH₂)ₜ- où t vaut 2 ou 3, et R² représente un groupement 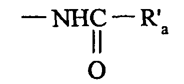 où R'ₐ représente un groupement alkyle, polyhalogénoalkyle, alkényle, alkynyle, cycloakyle, cycloalkényle, cycloakylalkyle, cycloalkénylalkyle, aryle, arylalkyle, hétéroaryle ou hétéroarylalkyle, comme par exemple méthyle, éthyle, propyle, isopropyle, butyle, isobutyle, secbutyle, tertbutyle, pentyle, isopentyle, hexyle, trifluorométhyle, vinyle, allyle, propargyle, phényle, naphtyle, benzyle, phénéthyle, cyclopropyle, cyclobutyle, cyclopentyle, cyclohexyle, cyclohexényle, méthylcyclopropyle, éthylcyclopropyle, furyle, thiényle, pyridyle, furylméthyle, pyridylméthyle,
ou G représente un groupement -(CH₂)₃- et R² représente un groupement 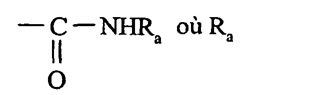 représente un groupement alkyle, polyhalogénoalkyle, alkényle, alkynyle, cycloakyle, cycloalkényle, cycloakylalkyle, cycloalkénylalkyle, aryle, arylalkyle, hétéroaryle ou hétéroarylalkyle, comme par exemple méthyle, éthyle, propyle, isopropyle, butyle, isobutyle, secbutyle, tertbutyle, pentyle, isopentyle, hexyle, trifluorométhyle, vinyle, allyle, propargyle, phényle, naphtyle, benzyle, phénéthyle, cyclopropyle, cyclobutyle, cyclopentyle, cyclohexyle, cyclohexényle, méthylcyclopropyle, éthylcyclopropyle, furyle, thiényle, pyridyle, furylméthyle, pyridylméthyle.

Plus avantageusement, l'invention concerne les composés pour lesquels :
A représente un groupement de formule (II) ou (III),
et R représente un groupement NR'ₐR"ₐ (où R'ₐ et R"ₐ sont tels que définis précédemment).

De façon encore plus avantageuse, les composés préférés de l'invention sont ceux pour lesquels
A représente un système cyclique de formule (II) ou (III),
R représente un groupement NR'ₐR"ₐ (où Rₐ' et R"ₐ sont définis comme précédemment),
et R' est tel que G représente une chaîne alkylène -(CH₂)ₜ- où t vaut 2 ou 3, et R² représente un groupement 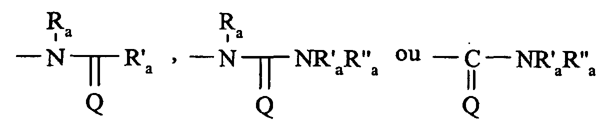 où Q, Rₐ, R'ₐ et R"ₐ sont tels que définis précédemment.

Encore plus particulièrement, l'invention concerne les (dihydro)benzothiophène, (dihydro)benzofurane, indole, indoline, indène, indane, azaindole, thiéno ou furopyridine éventuellement substitués en position 2, et les dihydronaphtalène, tétrahydronaphtalène, naphtalène ou chromane éventuellement substitués en position 3,
substitués en 5 (7 respectivement) par un groupement -NR'ₐR"ₐ où R'ₐ et R"ₐ, identiques ou différents représentent un atome d'hydrogène, un groupement alkyle, polyhalogénoalkyle, alkényle, alkynyle, cycloalkyle, cycloalkényle, cycloalkylalkyle, cycloalkénylakyle, aryle, arylalkyle, hétéroaryle ou hétéroarylalkyle, comme par exemple méthyle, éthyle, propyle, isopropyle, butyle, isobutyle, secbutyle, tertbutyle, pentyle, isopentyle, hexyle, trifluorométhyle, vinyle, allyle, propargyle, phényle,' naphtyle, benzyle, phénéthyle, cyclopropyle, cyclobutyle, cyclopentyle, cyclohexyle, cyclohexényle, méthylcyclopropyle, éthylcyclopropyle, furyle, thiényle, pyridyle, furylméthyle, pyridylméthyle, ou R'ₐ et R''ₐ forment, avec l'atome d'azote qui les porte un groupement piperazine, piperidine, morpholine ou thiomorpholine,
et substitués en 3 (1 respectivement) par un groupement -(CH₂)ₜ-NHCOR'ₐ où t vaut 2 ou 3 et R'ₐ représente un groupement alkyle, polyhalogénoalkyle, alkényle, alkynyle, cycloalkyle, cycloalkényle, cycloalkylalkyle, cycloalkénylalkyle, aryle, arylalkyle, hétéroaryle ou hétéroarylalkyle, comme par exemple méthyle, éthyle, propyle, isopropyle, butyle, isobutyle, secbutyle, tertbutyle, pentyle, isopentyle, hexyle, trifluorométhyle, vinyle, allyle, propargyle, phényle, naphtyle, benzyle, phénéthyle, cyclopropyle, cyclobutyle, cyclopentyle, cyclohexyle, cyclohexényle, méthylcyclopropyle, éthyle cyclopropyle, furyle, thiényle, pyridyle, furylméthyle, pyridylméthyle.

Encore plus avantageusement, les composés préférés de l'invention sont des naphtalènes éventuellement substitués en position 3, substitués en 7 par un groupement thioalkyle comme par exemple thiométhyle, thioéthyle, thiopropyle, et substitués en 1 par un groupement -(CH₂)ₜ-NHCOR'ₐ où t vaut 2 ou 3 et R'ₐ représente un groupement alkyle, polyhalogénoalkyle, cycloalkyle comme par exemple méthyle, éthyle, propyle, trifluorométhyle, cyclopropyle, cyclobutyle, cyclopentyle, cyclohexyle.

L'invention concerne tout particulièrement les composés de formule (I) qui sont les :
* N-{2-[7-Amino-3-(cyclopropylméthyl)-1-naphtyl]éthyl}acétamide
* N-{2-[7-(Diéthylamino)-1-naphtyl]éthyl}-2-phénylacétamide
* N-{2-[7-(Hexylamino)-1,2,3,4-tétrahydro-1-naphtalényl]éthyl}acétamide

Les énantiomères, diastéréoisomères ainsi que les sels d'addition à un acide ou une base pharmaceutiquement acceptable des composés préférés de l'invention font partie intégrale de l'invention.

L'invention s'étend également au procédé de préparation des composés de formule (I) caractérisé en ce que l'on utilise comme produit de départ le composé de formule (VIII) : 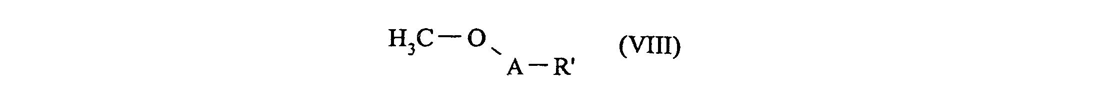 dans laquelle A et R' sont tels que définis précédemment, que l'on soumet à une déméthylation en utilisant des agents classiques comme HBr, AlCl₃, AlBr₃, BBr₃ ou des systèmes binaires acides de Lewis/nucléophiles comme AlCl₃/PhCH₂SH, ou BBr₃/Me₂S par exemple, pour obtenir le composé de formule (IX) :

HO―A―R' (IX)

dans laquelle A et R' sont définis comme précédemment,
que l'on transforme, grâce à l'action de réactifs tels que POCl₃, PCl₅, Ph₃PBr₂, PhPCl₄, HBr ou HI en dérivé halogéné correspondant de formule (XX) :

Hal―A-R' (XX)

dans laquelle A et R' sont définis comme précédemment et Hal représente un atome d'halogène (les composés de formule (XX) pouvant être obtenus par des réactions d'échange comme par exemple le traitement d'un dérivé chloré par KF au sein de la diméthylformamide pour conduire au dérivé fluoré correspondant, ou le traitement d'un dérivé bromé par KI en présence de sels de cuivre pour conduire au dérivé iodé correspondant, composés de formule (XX) pouvant par ailleurs être obtenus à partir de composés de formule (XX₁) ou (XX₂) : 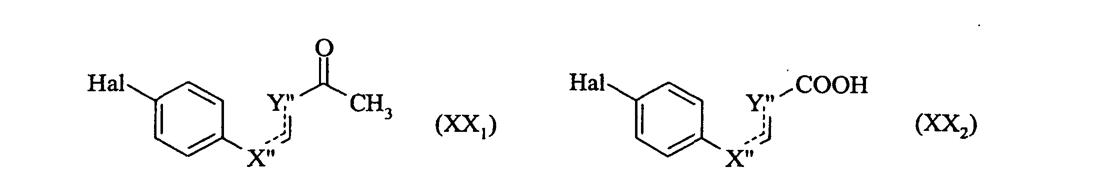 dans lesquels Hal est défini comme précédemment, X" représente un atome d'oxygène ou de soufre, un groupement C(H)_{q} (où q vaut 0, 1 ou 2) ou NR₀ (où R₀ est tel que défini précédemment), et Y" représente une liaison ou un groupement C(H)_{q} (où q vaut 0, 1 ou 2)),
composé de formule (XX) qui est :
- soit traité par du monoxyde de carbone et Bu₃SnH, la réaction étant catalysée par du palladium (0), pour conduire à l'aldéhyde correspondant de formule (XXI) : 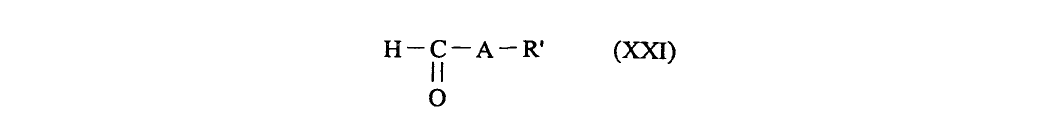 dans laquelle A et R' sont tels que définis précédemment,
   composé de formule (XXI) pouvant par ailleurs être obtenu par des techniques classiques de lithiations à partir du dérivé halogéné de formule (XX), ou par l'intermédiaire du dérivé
   vinylique correspondant (obtenu à partir du composé de formule (XX) par action de vinyltributylétain et de palladium tetrakis) soumis à une ozonolyse, ou encore par formylation directe du noyau A selon une réaction de Vilsmeier par exemple,
   composé de formule (XXI) que l'on soumet à un agent oxydant pour obtenir le composé de formule (XXII) :

   HOOC―A-R' (XXII)

   dans laquelle A et R' sont définis comme précédemment, qui est transformé, après l'action de chlorure de thionyle et d'un azidure, puis d'un acide, en composé de formule (I/g), cas particulier des composés de formule (I) :

   H₂N―A-R' (I/g)

   dans laquelle A et R' sont définis comme précédemment, sur lequel on condense une ou deux molécules d'un composé de formule (XVIII) :

   R₁ₐ―J (XVII)

   dans laquelle R₁ₐ peut prendre toutes les valeurs du groupement Rₐ tel que défini précédemment à l'exception de l'atome d'hydrogène et J représente un groupe partant comme un atome d'halogène ou un groupement tosyle,
   pour obtenir le composé de formule (I/h), cas particulier des composés de formule (I) :

   R'₂ₐR₂ₐN―A―R' (I/h)

   dans laquelle A et R' ont la même signification que précédemment et R'₂ₐ et R₂ₐ, identiques ou différents, représentent un groupement Rₐ avec la condition suivante : R'₂ₐ et R₂ₐ ne peuvent représenter simultanément un atome d'hydrogène et ne peuvent former, avec l'atome d'azote qui les porte un groupement cyclique,
- ou composé de formule (XX) qui est soumis, dans des conditions de substitution nucléophile aromatique, à l'action d'une amine R'ₐR''ₐNH dans laquelle R'ₐ et R''ₐ sont tels que définis précédemment (R'ₐ et R''ₐ peuvent entre autres former avec l'atome d'azote qui les porte un groupement cyclique tel que défini précédemment), pour conduire au composé de formule (I/i), cas particulier des composés de formule (I) :

   R'ₐR"ₐN―A―R' (I/i)
dans laquelle R'ₐ, R"ₐ, A et R' sont tels que définis précédemment,
les composés (I/g) à (I/i) pouvant être purifiés selon une technique classique de séparation, que l'on transforme, si on le souhaite, en leurs sels d'addition à un acide ou à une base pharmaceutiquement acceptable, et dont on sépare éventuellement les isomères selon une technique classique de séparation.
Les composés (VIII) de départ sont soit commerciaux, soit décrits dans la littérature comme par exemple dans les demandes EP0447285, EP0527687, EP0562956, EP0591057, EP0662471, EP0745586, EP0709371, EP0745583, EP0721938, EP0745584, EP0737670, EP0737685, ou WO9738682.

Les composés de l'invention et les compositions pharmaceutiques les contenant s'avèrent être utiles pour le traitement des troubles du système mélatoninergique.

L'invention concerne également les composés de formule (XX_{A}), cas particulier des composés de formule (XX) :

Hal―A_{A}―R'_{A} (XXA)

dans laquelle :
◆ Hal représente un atome d'halogène (fluor, chlore, brome, iode)
◆ A_{A} représente :
   - un cycle de formule (a) : 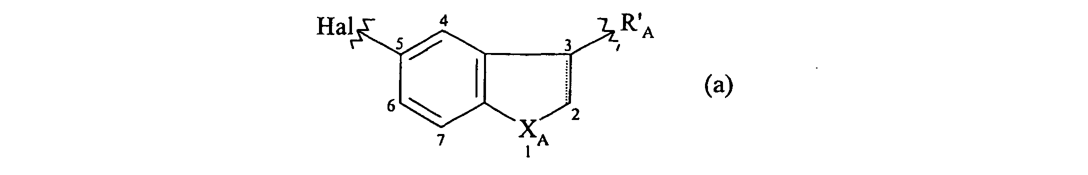 dans laquelle X_{A} représente un atome de soufre, ou un groupement NR₀ (dans lequel R₀ est tel que défini précédemment), et la représentation .... a la même signification que précédemment,
   - ou un cycle de formule (b) : 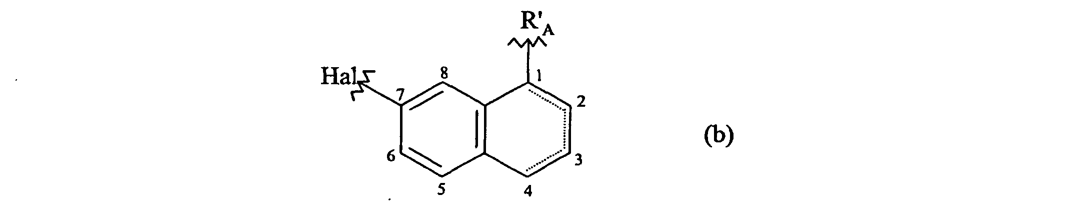 dans laquelle la représentation .... a la même signification que précédemment,
      les cycles de formule (a) ou (b) pouvant être substitués (en plus de l'atome d'halogène et du groupement R'_{A}) en position 2 (formule (a)) ou 3 (formule (b)) par un ou plusieurs groupements choisis parmi Rₐ, CORₐ, COORₐ, OCORₐ où Rₐ est tel que défini précédemment,
◆ et R'_{A} représente un groupement G-R²_{A} dans lequel G est tel que défini précédemment et R²_{A} représente un groupement 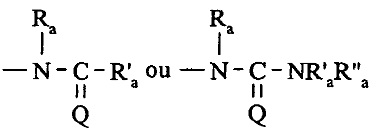 où Rₐ, R'ₐ, R"ₐ et Q sont tels que définis précédemment,
   leurs énantiomères et diastéréoisomères, ainsi que leurs sels d'addition à un acide ou une base pharmaceutiquement acceptable,
   en tant qu'intermédiaires de synthèse mais également en tant que composés utiles pour le traitement des troubles liés au système mélatoninergique.

L'étude pharmacologique des dérivés de l'invention a en effet montré qu'ils étaient atoxiques, doués d'une haute affinité pour les récepteurs de la mélatonine et possédaient d'importantes activités sur le système nerveux central et, en particulier, on a relevé des propriétés thérapeutiques sur les troubles du sommeil, des propriétés anxiolytiques, antipsychotiques, analgésiques ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des pathologies du système digestif, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention peuvent être utilisés dans les dysfonctionnements sexuels, qu'ils possèdent des propriétés d'inhibiteurs de l'ovulation, d'immunomodulateurs et qu'ils sont susceptibles d'être utilisés dans le traitement des cancers.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en 1 ou plusieurs prises.

Les exemples suivants illustrent l'invention et ne la limitent en aucune façon. Les préparations suivantes conduisent à des composés de l'invention ou à des intermédiaires de synthèse utiles dans la préparation des composés de l'invention.

### Préparation 2 : N-[2-(7-Hydroxy-1-naphtyl)éthyl]acétamide

Sous atmosphère inerte, 27,5 mmol de complexe tribromure de bore/diméthylsulfure sont dissoutes dans 100 ml de dichlorométhane et agitées pendant 15 min à température ambiante. Une solution de 13,7 mmol de N-[2-(7-méthoxy-1-naphtyl)éthyl]acétamide dans 50 ml de dichlorométhane est ajoutée, et le milieu réactionnel est porté à reflux pendant 30 heures. Après refroidissement, la réaction est hydrolysée avec précaution et le dichlorométhane est évaporé. Le milieu est alors extrait à l'acétate d'éthyle, les phases organiques regroupées sont lavées par une solution aqueuse de bicarbonate de potassium 1M, puis par une solution de soude 1M. La phase organique est séchée sur sulfate de magnésium, et concentrée pour conduire au composé du titre.

### Préparation 3 : N-[2-(7-Hydroxy-1-naphtyl)éthyl]-2-phénylacétamide

On procède comme dans la Préparation 2, mais en remplaçant le N-[2-(7-méthoxy-1-naphtyl) éthyl]acétamide par le N-[2-(7-méthoxy-1-naphtyl)éthyl]-2-phénylacétamide.

Dans les préparations 4 à 125, on procède comme dans la préparation 2, mais en remplaçant le N-[2-(7-méthoxy-1-naphtyl) éthyl]acétamide par le substrat de départ méthoxylé approprié.

### Préparation 8 : N-[2-(7-Hydroxy-1-naphtyl)éthyl]-2-bromoacétamide

### Préparation 20 : N-Cyclopropylméthyl-2-(7-hydroxy-1-naphtyl)acétamide

### Préparation 21 : N-Cyclohexyl-4-(7-hydroxy-1-naphtyl)butanamide

### Préparation 25 : N-[2-(7-Hydroxy-1-naphtyl)éthyl]-N-méthyl-N'-propylurée

### Préparation 33 : N-[3-(7-Hydroxy-1-naphtyl)propyl]acétamide

### Préparation 34 : N-[3-(7-Hydroxy-1-naphtyl)propyl]-1-cyclohexane carboxamide

### Préparation 42 : N-[2-(3-Benzoyl-7-Hydroxy-1-naphtyl)éthyl]-N'-propylurée

### Préparation 50 : N-{2-[3-(Cyclopropylméthyl)-7-hydroxy-1-naphtyl]éthyl}acétamide

### Préparation 52 : N-[3-(5-Hydroxybenzo[b]furan-3-yl)propyl]acétamide

### Préparation 54 : N-Méthyl-4-(5-Hydroxybenzo[b]furan-3-yl)butanamide

### Préparation 59 : N-[(2-Benzyl-5-hydroxybenzo[b]thiophen-3-yl)méthyl]acétamide

### Prénaration 64 : N-[2-(5-Hydroxy-1H-3-indolyl)éthyl]-2-morpholinoacétamide

### Préparation 66 : N-[2-(5-Hydroxy-1H-3-indolyl)éthyl]benzamide

### Préparation 122 : N-{2-[7-Hydroxy-3-naphtyl-1-naphtyl]éthyl}heptanamide

### Préparation 124 : N-{2-[7-Hydroxy-3-(3-phényl-2-propényl)-1-naphtyl]éthyl}-2-phénylacétamide

### Préparation 134 : N-[2-(7-Bromo-1-naphtyl)éthyl]-2-phénylacétamide

Dans un tricol de 150 ml équipé d'une ampoule à brome, d'un réfrigérant surmonté d'un tube rempli de chlorure de calcium, et d'un agitateur mécanique, on verse de la triphénylphosphine (10 mmol) et de l'acétonitrile (70 ml). La solution est refroidie à l'aide d'un bain de glace en maintenant l'agitation et on additionne le brome (10 mmol). A la fin de l'addition, le bain de glace est retiré puis on ajoute le produit obtenu dans la Préparation 3 (8 mmol). Le mélange réactionnel est agité à 60-70°C jusqu'à disparition du produit de départ (suivi par CCM). En fin de réaction, le mélange est filtré puis le filtrat est concentré sous pression réduite. Le résidu est repris dans l'acétate d'éthyle, lavé à l'eau puis avec une solution saturée d'hydrogénocarbonate de potassium, et encore une fois à l'eau puis séché sur sulfate de magnésium et concentré sous pression réduite. Le résidu est filtré sur gel de silice pour conduire au produit du titre.

Dans les préparations 135 à 159, on procède comme dans la Préparation 134 en partant du réactif approprié.

### Préparation 136 : N-Cyclopropylméthyl-2-(7-bromo-1-naphtyl)acétamide

*Produit de départ : Préparation 20*

### Préparation 137 : N-[2-(7-Bromo-1-naphtyl)éthyl]-N-méthyl-N'-propylurée

*Produit de départ : Préparation 25*

### Préparation 139 : N-[3-(7-Bromo-1-naphtyl)propyl]-1-cyclohexane carboxamide

*Produit de départ : Préparation 34*

### Préparation 141 : N-[2-(3-Benzoyl-7-bromo-1-naphtyl)éthyl]-N'-propylurée

*Produit de départ : Préparation 42*

### Préparation 142 : N-[3-(5-Bromobenzo[b]furan-3-yl)propyl]acétamide

*Produit de départ : Préparation 52*

### Préparation 143 : N-[(2-Benzyl-5-bromobenzo[b]thiophèn-3-yl)méthyl]acétamide

*Produit de départ : Préparation 59*

### Préparation 145 : N-[2-(5-Bromo-1H-3-indolyl)éthyl]-2-morpholinoacétamide

*Produit de départ : Préparation 64*

### Préparation 158 :-N-[2-(7-Bromo-3-naphtyl)éthyl]heptanamide

*Produit de départ : Préparation 122*

### Préparation 159 : N-{2-[7-Bromo-3-(3-phényl-2-propényl)-1-napthyl])éthyl}-2-cyclohexyl acétamide

*Produit de départ : Préparation 124*

### Préparation 160 : N-[2-(7-Iodo-1-naphtyl)éthyl]-2-phénylacétamide

Un mélange du produit obtenu dans la Préparation 134 (2 mmol), d'iodure de potassium (30 mmol) et d'iodure de cuivre 1 (10 mmol) dans l'hexaméthyl phosphoramide (6 ml) est chauffé à 150-160°C avec agitation sous atmosphère d'azote jusqu'à ce qu'un taux de conversion de 90 % soit atteint (suivi en CCM). On ajoute alors de l'acide chlorhydrique dilué puis de l'éther et la mixture est alors filtrée pour éliminer les sels de cuivre (I) insolubles. La phase organique est séparée, lavée avec une solution de sulfite de sodium, de l'eau, séchée sur sulfate de magnésium et évaporée pour donner un résidu que l'on chromatographie sur gel de silice pour conduire au produit du titre.

Dans les préparations 161 à 185, on procède comme dans la Préparation 160, mais en remplaçant le produit de la Préparation 134 par le substrat approprié.

### Préparation 162 : N-Cyclopropylméthyl-2-(7-iodo-1-naphtyl)acétamide

*Produit de départ : Préparation 136*

### Préparation 163 : N-[2-(7-Iodo-1-naphtyl)éthyl]-N-méthyl-N'-propylurée

*Produit de départ : Préparation 137*

### Préparation 165 : N-[3-(7-Iodo-1-naphtyl)propyl]-1-cyclohexanecarboxamide

*Produit de départ : Préparation 139*

### Préparation 167 : N-[2-(3-Benzoyl-7-iodo-1-naphtyl)éthyl]-N'-propylurée

*Produit de départ : Préparation 141*

### Préparation 168 : N-[3-(5-Iodobenzo[b]furan-3-yl)propyl]acétamide

*produit de départ : Préparation 142*

### Préparation 169 : N-[(2-Benzyl-5-iodobenzo[b]thiophen-3-yl)méthyl]acétamide

*Produit de départ : Préparation 143*

### Préparation 171 : N-[2-(5-Iodo-1H-3-indolyl)éthyl]-2-morpholinoacétamide

*Produit de départ : Préparation 145*

### Préparation 184 : N-[2-(7-Iodo-3-napthyl-1-naphtyl)éthyl]heptanamide

*Produit de départ : Préparation 158*

### Préparation 185 : N-{2-[7-Iodo-3-(3-phényl-propényl)-1-naphtyl]éthyl}-2-cyclohexyl acétamide

*Produit de départ : Préparation 159*

Dans les préparations 186 à 197, on procède comme dans la Préparation 134, à partir du substrat approprié.

### Préparation 186 : N-[2-(7-Bromo-1-naphtyl)éthyl]-2-bromoacétamide

*Produit de départ : Préparation 8*

### Préparation 188 : N-Cyclohexyl-4-(7-bromo-1-naphtyl)butanamide

*Produit de départ : Préparation 21*

### Préparation 189 : N-[3-(7-Bromo-1-naphtyl)propyl]acétamide

*Produit de départ : Préparation 33*

### Préparation 191 : N-{2-[7-Bromo-3-(cyclopropylméhyl)-1-naphtyl)éthyl}acétamide

*Produit de départ : Préparation 50*

### Préparation 192 : N-Méthyl-3-(5-bromobenzo[b]furan-3-yl)butanamide

*Produit de départ : Préparation 54*

### Préparation 194 : N-[2-(5-Bromo-1H-3-indolyl)éthyl]benzamide

*Produit de départ : Préparation 66*

Dans les préparations 198 à 209, on procède comme dans la Préparation 160, à partir du substrat approprié.

### Préparation 198 : N-[2-(7-Iodo-1-naphtyl)éthyl]-2-bromoacétamide

*Produit de départ : Préparation 186*

### Préparation 200 : N-Cyclohexyl-4-(7-Iodo-1-naphtyl)butanamide

*Produit de départ : Préparation 188*

### Préparation 201 : N-[3-(7-Iodo-1-naphtyl)propyl]acétamide

*Produit de départ : Préparation 189*

### Préparation 203 : N-{2-[7-Iodo-3-(cyclopropylméthyl)-1-naphtyl]éthyl}acétamide

*Produit de départ : Préparation 191*

### Préparation 204 : N-Méthyl-4-(5-iodobenzo[b]furan-3-yl)butanamide

*Produit de départ : Préparation 192*

### Préparation 206 : N-[2-(5-Iodo-1H-3-indolyl)éthyl]benzamide

*Produit de départ : Préparation 194*

### Préparation 244 : N-[2-(7-Iodo-1,2,3,4-tétrahydro-1-naphtalényl)éthyl]acétamide

On procède comme dans la Préparation 160.

### Préparation 246 : N-[2-(5-Iodobenzo[b]furan-3-yl)éthyl]acétamide

On procède comme dans la Préparation 160.

### EXEMPLE 268 : N-[2-(7-Amino-1-naphtyl)éthyl]-2-phénylacétamide

### Stade A : N-[2-(7-Vinyl-1-naphtyl)éthyl]-2-phénylacétamide

15 mmol du produit obtenu dans la Préparation 160, 16 mmol de vinyl tributylétain et 0,43 mmol de (triphénylphosphine) palladium tetrakis, sont portés sous agitation à 110°C pendant 3 heures dans 30 mL de N-méthylpyrrolidinone. Après évaporation du solvant, le résidu est repris dans 20 mL de dichlorométhane et traité par une solution aqueuse 10 % de fluorure de potassium. Après extraction, concentration sous pression réduite et chromatographie sur gel de silice, on obtient le produit du titre pur.

### Stade B : N-[2-(7-Formyl-1-naphtyl)éthyl]-2-phénylacétamide

A une solution de 10 mmol du produit obtenu dans le stade A dans un mélange de 50 mL de dioxane et 25 mL d'eau est ajouté à température ambiante 1,10 g de tétroxyde d'osmium dans le 2-méthyl-2-propanol, puis 8,70 g de Periodate de sodium. Après agitation une nuit à température ambiante, la suspension est filtrée, le filtrat concentré sous pression réduite. Le résidu obtenu est repris dans le dichlorométhane. La phase organique est lavée avec de l'eau, séchée et évaporée. Le résidu est purifié par chromatographie sur gel de silice pour conduire au produit du titre.

### Stade C : Acide 8-{2-[(2-Phénylacétyl)amino]éthyl}-2-naphtoïque

A une solution de 6,88 mmol du produit obtenu dans le stade B dans 30 mL d'acétone sont ajoutés à température ambiante 2,7 g de permanganate de potassium dans 50 mL d'un mélange acétone/eau (50/50). La solution est agitée 2 heures à température ambiante puis filtrée. Le filtrat est concentré sous pression réduite et chromatographié sur gel de silice pour conduire au produit du titre.

### Stade D : Chlorure de 8-{2-[(2-phénylacétyl)amino]éthyl}-2-napthtalènecarbonyle

5 mmol du produit obtenu dans le stade C sont dissoutes dans 40 mL de chlorure de thionyle. Après agitation sous atmosphère inerte pendant 1 heure, le chlorure de thionyle est évaporé sous pression réduite pour conduire au produit du titre.

### Stade E : N-[2-(7-Amino-1-naphtyl)éthyl]-2-phénylacétamide

Une solution du produit obtenu dans le stade D (20 mmol) dans le dichlorométhane (30 mL) contenant du bromure de tétrabutyl ammonium (20 mg) est refroidie dans un bain de glace. Après addition de l'azoture de sodium (24 mmol) dissous dans 5 ml d'eau , la solution est agitée vigoureusement à 0°C pendant 2 heures. La phase organique est séparée, lavée à l'eau (2 x 5 ml) et séchée sur sulfate de magnésium. Après filtration, on ajoute l'acide trifluoroacétique (30 mmol) et la solution est agitée sous reflux pendant 60 heures. Après refroidissement, la phase organique est lavée avec une solution saturée d'hydrogénocarbonate de sodium (2 x 5 mL) et concentrée sous pression réduite. Le résidu est alors repris dans le méthanol (20 mL) et on ajoute de l'eau (80 mL) puis du carbonate de potassium (30 mmol). Après agitation à température ambiante pendant 20 heures, le milieu réactionnel est concentré sous pression réduite jusqu'à un volume de 60 mL environ puis extrait 3 fois à l'éther (3 x 50 mL). Après séchage sur sulfate de sodium, la phase organique est filtrée puis évaporée sous pression réduite. Le résidu est chromatographié sur gel de silice pour conduire au produit du titre.

Dans les exemples 269 à 289, on procède comme dans l'exemple 268 en partant du substrat approprié.

### EXEMPLE 269 : N-[2-(7-Amino-1-naphtyl)éthyl]-2-bromoacétamide

*Produit de départ : Préparation 198*

### EXEMPLE 271 : N-Cyclohexyl-4-(7-amino-1-naphtyl)butanamide

*Produit de départ : Préparation 200*

### EXEMPLE 272 : N-[3-(7-Amino-1-naphtyl)propyl]acétamide

*Produit de départ : Préparation 201*

### EXEMPLE 274 : N-[2-(7-Amino-3-benzoyl-1-naphtyl)éthyl]-N'-propylurée

*Produit de départ : Préparation 167*

### EXEMPLE 275 : N-{2-[7-Amino-3-(cyclopropylméthyl)-1-naphtyl]éthyl}acétamide

*Produit de départ : Préparation 203*

### EXEMPLE 276 : N-Méthyl-4-(5-aminobenzo[b]furan-3-yl)butanamide

*Produit de départ : Préparation 204*

### EXEMPLE 278: N-[2-(5-Amino-1H-3-indolyl)éthyl]benzamide

*Produit de départ : Préparation 206*

### EXEMPLE 280 : N-[2-(5-Amino-2-benzylbenzo[b]furan-3-yl)éthyl]-1-cyclopropane carboxamide

*Produit de départ : Préparation 207*

### EXEMPLE 286 : N-[2-(7-Amino-3-phényl-1-naphtyl)éthyl]acétamide

*Produit de départ : Préparation 243*

### EXEMPLE 289 : N-[2-(7-Amino-3-naphtyl-1-naphtyl)éthyl]heptanamide

*Produit de départ : Préparation 184*

### EXEMPLE 290 : N-{2-(7-(Diéthylamino)-1-naphtyl]éthyl}-2-phénylacétamide

A une solution du produit de la Préparation 160 (5 mmol), de diéthylamine (12 mmol) et de tert-butoxyde de sodium (14 mmol), dans le dioxane (20 ml), on ajoute du tris(dibenzylidèneacétone) dipalladium (0,25 mmol, 1 % molaire de Palladium) et de la tri-o-tolylphosphine (0,1 mmol). On chauffe ensuite à 100°C avec agitation jusqu'à consommation complète du produit de départ (suivi par HPLC). On refroidit alors la solution à température ambiante et on ajoute 150 ml d'éther. La phase organique est lavée avec de la saumure (75 ml) puis séchée sur sulfate de magnésium, filtrée et concentrée sous pression réduite. Le résidu est alors chromatographié sur gel de silice pour conduire au produit du titre.

Dans les exemples 291 à 315, on procède comme dans l'Exemple 290 à partir de la Préparation appropriée.

### EXEMPLE 292 : N-Cyclopropylméthyl-2-[7-(3,5-diméthylpipérazino)-1-naphtyl] acétamide

*Produit de départ : Préparation 162*

### EXEMPLE 293 : N-Méthyl-N-{2-[7-(méthylanilino)-1-naphtyl]éthyl}-N'-propylurée

*Produit de départ : Préparation 163*

### EXEMPLE 295 : N-{3-[7-(Benzyl[1-éthynyl]amino)-1-naphtyl]propyl}-1-cyclohexane carboxamide

*Produit de départ : Préparation 165*

### EXEMPLE 296 : N-{2-[7-(Hexylamino)-1,2,3,4-tétrahydro-1-naphtalényl]éthyl}acétamide

*Produit de départ : Préparation 244*

### EXEMPLE 297 : N-{2-[3-Benzoyl-7-(propylamino)-1-naphtyl]éthyl}-N'-propylurée

*Produit de départ : Préparation 167*

### EXEMPLE 298 : N-{3-[5-(Hexyl[2-propynyl]amino)benzo[b]furan-3-yl]propyl}acétamide

*Produit de départ : Préparation 168*

### EXEMPLE 299 : N-{(2-Benzyl-5-([1-éthyl-2-propynyl]amino)benzo[b]thiophèn-3-yl] méthyl}acétamide

*Produit de départ : Préparation 169*

### EXEMPLE 301 : N-[2-(5-Phénylamino-1H-3-indolyl)éthyl]-2-morpholinoacétamide

*Produit de départ : Préparation 171*

### EXEMPLE 314 : N-{2-[7-(3,5-Diméthylpipérazino)-3-naphtyl-1-naphtyl]éthyl} heptanamide

*Produit de départ : Préparation 184*

### EXEMPLE 315 : N-{2-[3-Phényl-2-propényl)-7-[(3-phényl-2-propényl]amino)-1-naphtyl] éthyl}-2-cyclohexylacétamide

*Produit de départ : Préparation 185*

Les Exemples 350 à 353 sont obtenus en procédant comme dans l'Exemple 268 à partir des substrats appropriés.

### EXEMPLE 350 : N-[2-(5-Aminobenzo[b]furan-3-yl)éthyl]acétamide

*Produit de départ : Préparation 246*

### EXEMPLE 351 : N-[2-(7-Amino-1,2,3,4-tetrahydro-1-naphtalényl)éthyl]acétamide

*Produit de départ : Préparation 244*

### EXEMPLE 353 : N-{2-[5-(Méthylamino)benzo[b]furan-3-yl)éthyl]acétamide

On procède comme dans l'Exemple 290 à partir de la Préparation 246.

### ETUDE PHARMACOLOGIQUE

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Etude de liaison aux récepteurs de la mélatonine sur des cellules de la pars tuberalis de mouton

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology, 1, pp. 1-4, 1989).

### Protocole

1) Les membranes de pars tuberalis de mouton sont préparées et utilisées comme tissu cible dans des expériences de saturation pour déterminer les capacités et affinités de liaison pour la 2-[¹²⁵I]-iodomélatonine.
2) Les membranes de pars tuberalis de mouton sont utilisées comme tissu cible, avec les différents composés à tester, dans des expériences de liaison compétitive par rapport à la mélatonine.

Chaque expérience est réalisée en triple et une gamme de concentrations différentes est testée pour chaque composé. Les résultats permettent de déterminer, après traitement statistique, les affinités de liaison du composé testé.

### Résultats

Il apparaît que les composés de l'invention possèdent une puissante affinité pour les récepteurs de la mélatonine.

### EXEMPLE C : Etude de liaison aux récepteurs mt₁ et MT₂ de la mélatonine

Les expériences de liaison aux récepteurs mt₁ ou MT₂ sont réalisées en utilisant la 2-[¹²⁵I]-iodomélatonine comme radioligand de référence. La radioactivité retenue est déterminée à l'aide d'un compteur à scintillation liquide.

Des expériences de liaison compétitive sont ensuite réalisées en triple, avec les différents composés à tester. Une gamme de concentrations différentes est testée pour chaque composé. Les résultats permettent de déterminer les affinités de liaison des composés testés (IC₅₀).

Ainsi, les valeurs d'IC₅₀ trouvées pour les composés de l'invention attestent d'une liaison pour l'un ou l'autre des sous-types réceptoriels mt₁ ou MT₂, ces valeurs étant ≤ 10 µM.

### EXEMPLE D : Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### Protocole expérimental

Des rats mâles âgés de un mois sont soumis dès leur arrivée au laboratoire à un cycle lumineux de 12h de lumière par 24h (LD 12 : 12).
Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats

Il apparaît clairement que les composés de l'invention permettent d'agir de façon puissante sur le rythme circadien via le système mélatoninergique.

### EXEMPLE E : Test des cages claires/obscures

Les composés de l'invention sont testés dans un modèle comportemental, le test des cages claires/obscures, qui permet de révéler l'activité anxiolytique des molécules.

L'appareil est composé de deux boîtes en polyvinyle couvertes de Plexiglas. L'une de ces boîtes est obscure. Une lampe est placée au-dessus de l'autre boîte donnant une intensité lumineuse au centre de celle-ci d'approximativement 4000 lux. Un tunnel opaque en plastique sépare la boîte claire de la boîte sombre. Les animaux sont testés individuellement pendant une session de 5 min. Le plancher de chaque boîte est nettoyé entre chaque session. Au début de chaque test, la souris est placée dans le tunnel, face à la boîte sombre. Le temps passé par la souris dans la boîte éclairée et le nombre de transitions à travers le tunnel sont enregistrés après la première entrée dans la boîte sombre.

Après administration des composés 30 min avant le début du test, les composés de l'invention augmentent de façon significative le temps passé dans la cage éclairée ainsi que le nombre des transitions, ce qui montre l'activité anxiolytique des dérivés de l'invention.

### EXEMPLE F : Activité des composés de l'invention sur l'artère caudale de rat

Les composés de l'invention ont été testés *in vitro* sur l'artère caudale de rat. Les récepteurs mélatoninergiques sont présents sur ces vaisseaux ce qui en fait un modèle pharmacologique relevant pour étudier l'activité de ligands mélatoninergiques. La stimulation des récepteurs peut induire soit une vasoconstriction soit une dilatation en fonction du segment artériel étudié.

### Protocole

Des rats âgés de 1 mois sont habitués pendant 2 à 3 semaines à un cycle lumière/obscurité 12h/12h.
Après sacrifice, l'artère caudale est isolée et maintenue dans un milieu richement oxygéné. Les artères sont ensuite cannulées aux deux extrémités, suspendues verticalement dans une chambre d'organe dans un milieu approprié et perfusées via leur extrémité proximale. Les changements de pression dans le débit de la perfusion permettent d'évaluer l'effet vasoconstricteur ou vasodilatateur des composés.
L'activité des composés est évaluée sur des segments pré-contractés par la phényléphrine (1 µM). Une courbe concentration-réponse est déterminée de façon non-cumulative par addition d'une concentration du composé étudié sur le segment pré-contracté. Lorsque l'effet observé a atteint l'équilibre, le milieu est changé et la préparation laissée 20 minutes avant l'addition d'une même concentration de phényléphrine et d'une nouvelle concentration du composé étudié.

### Résultats

Les composés de l'invention modifient de façon significative le diamètre des artères caudales préconstrictées par la phényléphrine.

### EXEMPLE G : Composition pharmaceutique : Comprimés

| | |
|---|---|
| 1000 comprimés dosés à 5 mg de N-{2-[7-Amino-3-(cyclopropylméthyl)-1-naphtyl]éthyl}acétamide (Exemple 275) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) :
R-A-R' (I)
dans laquelle :
◆ A représente :
- un système cyclique de formule (II) : 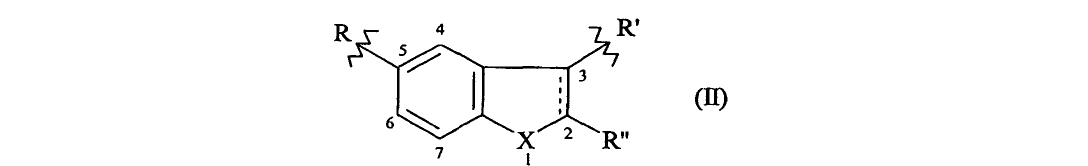 où
● X représente un atome d'oxygène ou de soufre, ou un groupement NR₀ (dans lequel R₀ représente un atome d'hydrogène, un groupement alkyle (C₁-C₆) linéaire ou ramifié, aryle, arylalkyle (C₁-C₆) linéaire ou ramifié ou SO₂Ph),
● la représentation .... signifie que la liaison peut être simple ou double étant entendu que la valence des atomes est respectée,
● R" représente un groupement Rₐ, ORₐ, CORₐ, COORₐ, OCORₐ, OSO₂CF₃, cyano, nitro ou atomes d'halogène,
où Rₐ représente un atome d'hydrogène, un groupement alkyle (C₁-C₆) linéaire ou ramifié substitué ou non, alkényle (C₂-C₆) linéaire ou ramifié substitué ou non, alkynyle (C₂-C₆) linéaire ou ramifié substitué ou non, polyhalogénoalkyle (C₁-C₆) linéaire ou ramifié, cycloalkyle (C₃-C₈) substitué ou non, cycloalkyle (C₃-C₈) alkyle (C₁-C₆) linéaire ou ramifié substitué ou non, cycloalkényle (C₃-C₈) substitué ou non, cycloalkényle (C₃-C₈) alkyle (C₁-C₆) linéaire ou ramifié substitué ou non, aryle, arylalkyle (C₁-C₆) linéaire ou ramifié, arylalkényle (C₁-C₆) linéaire ou ramifié, hétéroaryle, hétéroarylalkyle (C₁-C₆) linéaire ou ramifié, hétéroarylalkényle (C₁-C₆) linéaire ou ramifié, hétérocycloalkyle substitué ou non, hétérocycloalkényle substitué ou non, hétérocycloalkylalkyle (C₁-C₆) linéaire ou ramifié substitué ou non, ou hétérocycloalkényl alkyle (C₁-C₆) linéaire ou ramifié substitué ou non,
- ou un système cyclique de formule (III) : 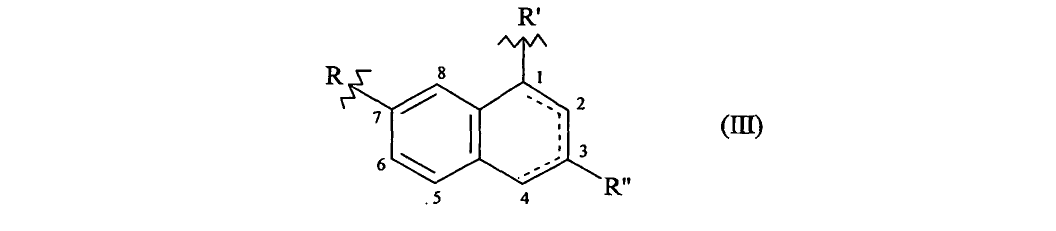 où R" et la représentation .... ont la même signification que précédemment,
◆ R représente :
un groupement -NR'ₐR"ₐ dans lequel R'ₐ et R"ₐ, identiques ou différents, peuvent prendre toutes les valeurs de Rₐ et peuvent également former, avec l'atome d'azote qui les porte un groupement cyclique contenant 5 à 10 chaînons pouvant comporter, en plus de l'atome d'azote, un à trois hétéroatomes choisis parmi oxygène, soufre et azote,
◆ et R' représente un groupement de formule (VII) :
―G―R² (VII)
où
● G représente une chaîne alkylène -(CH₂)ₜ- (dans laquelle t vaut 2 ou 3),
● et R² représente un groupement 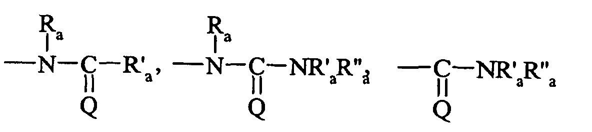 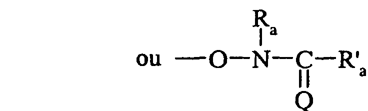
dans lesquels Q représente un atome d'oxygène ou de soufre, et Rₐ, R'ₐ et R"ₐ (identiques ou différents) sont définis de la même façon que précédemment, R'ₐ et R"ₐ pouvant former avec l'atome d'azote qui les porte un groupement cyclique tel que défini précédemment,
étant entendu que :
― par "hétérocycloalkyle" on entend tout groupement saturé mono ou polycyclique contenant de 5 à 10 atomes contenant 1 à 3 hétéroatomes choisis parmi azote, oxygène ou soufre,
― par "hétérocycloalkényle" on entend tout groupement mono ou polycyclique non aromatique contenant une ou plusieurs insaturations, contenant de 5 à 10 atomes et pouvant contenir 1 à 3 hétéroatomes choisis parmi azote, oxygène ou soufre,
― le terme "substitué" affecté aux expressions "alkyle", "alkényle", "alkynyle" signifie que ces groupements sont substitués par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, amino ou atomes d'halogène,
― le terme "substitué" affecté aux expressions "cycloalkyle", "cycloalkylalkyle", "cycloalkényle", "cycloalkénylalkyle", "hétérocycloalkyle", "hétérocyclo alkényle", "hétérocycloalkylalkyle", "hétérocycloalkénylalkyle", signifie que la partie cyclique de ces groupements est substituée par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, amino ou atomes d'halogène,
― par "aryle" on entend tout groupement aromatique mono ou polycyclique contenant 6 à 22 atomes de carbone, ainsi que le groupement biphényle,
― par "hétéroaryle" on entend tout groupement aromatique mono ou polycyclique contenant de 5 à 10 atomes contenant 1 à 3 hétéroatomes choisis parmi azote, oxygène ou soufre,
les groupements "aryle" et "hétéroaryle" pouvant être substitués par un ou plusieurs radicaux, identiques ou différents, choisis parmi hydroxy, alkoxy (C₁-C₆) linéaire ou ramifié, alkyle (C₁-C₆) linéaire ou ramifié, polyhalogénoalkyle (C₁-C₆) linéaire ou ramifié, cyano, nitro, amino ou atomes d'halogène,
étant entendu que :
- lorsque A représente un noyau indole substitué en position 2 par un phényle éventuellement substitué, alors R² ne peut représenter un groupement -NHCORₑ dans lequel Rₑ est un groupement contenant un hétérocycle mono ou bicyclique aromatique
ou non,
leurs énantiomères et diastéréoisomères, ainsi que leurs sels d'addition à un acide ou une base pharmaceutiquement acceptable."

2. Composés de formule (I) selon la revendication 1 tels que R' représente un groupement G-R² dans lequel G représente une chaîne alkylène -(CH₂)ₜ- où t vaut 2 ou 3, et R² représente un groupement -NHCOR'ₐ ou -CONHR'ₐ où R'ₐ est tel que défini dans la revendication 1, leurs énantiomères et diastéréoisomères, ainsi que leurs sels d'addition à un acide ou une base pharmaceutiquement acceptable.

3. Composés de formule (I) selon la revendication 1 tels que A représente un système cyclique de formule (II), leurs énantiomères et diastéréoisornères, ainsi que leurs sels d'addition à un acide ou une base pharmaceutiquement acceptable.

4. Composés de formule (I) selon la revendication 1 tels que A représente un système cyclique de formule (III), leurs énantiomères et diastéréoisomères, ainsi que leurs sels d'addition à un acide ou une base pharmaceutiquement acceptable.

5. Composés de formule (I) selon la revendication 1 tels que A représente un système cyclique de formule (II), et R' représente un groupement de formule (VII) où R² représente un groupement 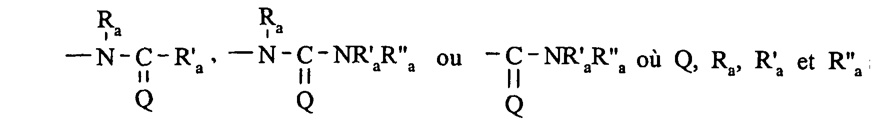 sont tels que définis dans la revendication 1, leurs énantiomères et diastéréoisomères, ainsi que leurs sels d'addition à un acide ou une base pharmaceutiquement acceptable.

6. Composés de formule (I) selon la revendication 1 tels que A représente un système cyclique de formule (III), et R' représente un groupement de formule (VII) où R² représente un groupement 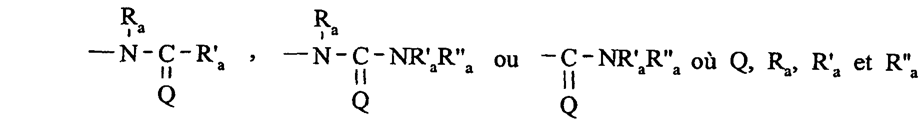 sont tels que définis dans la revendication 1, leurs énantiomères et diastéréoisomères, ainsi que leurs sels d'addition à un acide ou une base pharmaceutiquement acceptable.

7. Composés de formule (I) selon la revendication 1 tels que A représente un noyau naphtalène, dihydro ou tétrahydronaphtalène, leurs énantiomères et diastéréoisomères, ainsi que leurs sels d'addition à un acide ou une base pharmaceutiquement acceptable .

8. Composés de formule (I) selon la revendication 1 tels que A représente un noyau benzofurane ou dihydrobenzofurane, leurs énantiomères et diastéréoisomères, ainsi que leurs sels d'addition à un acide ou une base pharmaceutiquement acceptable.

9. Composés de formule (I) selon la revendication 1 tels que A représente un noyau benzothiophène ou dihydrobenzothiophène, leurs énantiomères et diastéréoisomères, ainsi que leurs sels d'addition à un acide ou une base pharmaceutiquement acceptable.

10. Composés de formule (I) selon la revendication 1 tels que A représente un noyau indole ou indoline, leurs énantiomères et diastéréoisomères, ainsi que leurs sels d'addition à un acide ou une base pharmaceutiquement acceptable.

11. Composés de formule (I) selon la revendication 1 tels que A représente un noyau naphtalène, dihydro ou tétrahydronaphtalène, et R' représente un groupement -(CH₂)ₜ-NHCOR'ₐ ou - (CH₂)ₜ-CONHR'ₐ où t vaut 2 ou 3, et R'ₐ est tel que défini dans la revendication 1, leurs énantiomères et diastéréoisomères, ainsi que leurs sels d'addition à un acide ou une base pharmaceutiquement acceptable.

12. Composés de formule (I) selon la revendication 1 tels que A représente un noyau benzofurane ou dihydrobenzofurane, et R' représente un groupement -(CH₂)ₜ-NHCOR_{'a} ou - (CH₂)ₜ-CONHR'ₐ où t vaut 2 ou 3, et R'ₐ est tel que défini dans la revendication 1, leurs énantiomères et diastéréoisomères, ainsi que leurs sels d'addition à un acide ou une base pharmaceutiquement acceptable.

13. Composés de formule (I) selon la revendication 1 tels que A représente un noyau benzothiophène ou dihydrobenzothiophène, et R' représente un groupement -(CH₂)ₜ-NHCOR'ₐ ou -(CH₂)ₜ-CONHR'ₐ où t vaut 2 ou 3, et R'ₐ est tel que défini dans la revendication 1, leurs énantiomères et diastéréoisomères, ainsi que leurs sels d'addition à un acide ou une base pharmaceutiquement acceptable.

14. Composés de formule (I) selon la revendication 1 tels que A représente un noyau indole ou indoline, et R' représente un groupement -(CH₂)ₜ-NHCOR'ₐ ou -(CH₂)ₜ-CONHR'ₐ où t vaut 2 ou 3, et R'ₐ est tel que défini dans la revendication 1, leurs énantiomères et diastéréoisomères, ainsi que leurs sels d'addition à un acide ou une base pharmaceutiquement acceptable.

15. Composés de formule (I) selon la revendication 1 qui sont :
* le N- {2-[7-Amino-3-(cyclopropylméthyl)-1-naphtyl]éthyl}acétamide,
* le N-{2-[7-(Diéthylamino)-1-naphtyl]éthyl}-2-phénylacétamide
* le N-{2-[7-(Hexylamino)-1,2,3,4-tétrahydro-1-naphtalényl]éthyl}acétamide
* le N-[(6-Morpholino-2-phényl-*2H*-3-chroményl)méthyl]acétamide,
leurs énantiomères et diastéréoisomères, ainsi que leurs sels d'addition à un acide ou une base pharmaceutiquement acceptable.

16. Procédé de préparation des composés de formule (I) selon la revendication 1 **caractérisé en ce que** l'on utilise comme produit de départ le composé de formule (VIII) : 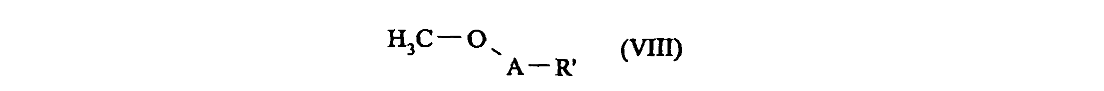 dans laquelle A et R' sont tels que définis précédemment, que l'on soumet à une déméthylation en utilisant des agents classiques comme HBr, AlCl₃, AlBr₃, BBr₃ ou des systèmes binaires acides de Lewis/nucléophiles comme AlCl₃/PhCH₂SH, ou BBr₃/Me₂S par exemple, pour obtenir le composé de formule (IX) :
HO―A―R' (IX)
dans laquelle A et R' sont définis comme précédemment,
que l'on transforme, grâce à l'action de réactifs tels que POCl₃, PCl₅, Ph₃PBr₂, PhPCl₄, HBr ou HI en dérivé halogéné correspondant de formule (XX) :
Hal―A-R' (XX)
dans laquelle A et R' sont définis comme précédemment et Hal représente un atome d'halogène (les composés de formule (XX) pouvant être obtenus par des réactions d'échange comme par exemple le traitement d'un dérivé chloré par KF au sein de la diméthylformamide pour conduire au dérivé fluoré correspondant, ou le traitement d'un dérivé bromé par KI en présence de sels de cuivre pour conduire au dérivé iodé correspondant, composés de formule (XX) pouvant par ailleurs être obtenus à partir de composés de formule (XX₁) ou (XX₂) : 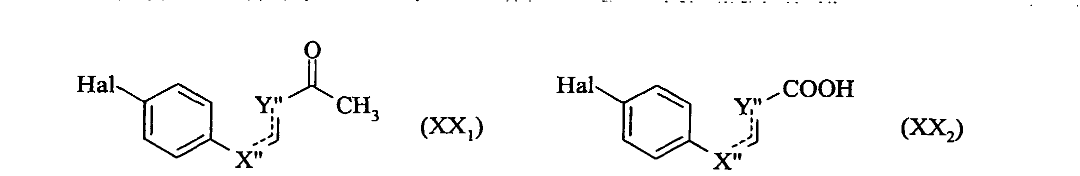 dans lesquels Hal est défini comme précédemment, X" représente un atome d'oxygène ou de soufre, un groupement C(H)_{q} (où q vaut 0, 1 ou 2) ou NR₀ (où R₀ est tel que défini précédemment), et Y" représente une liaison ou un groupement C(H)_{q} (où q vaut 0, 1 ou 2)),
composé de formule (XX) qui est :
• soit traité par du monoxyde de carbone et Bu₃SnH, la réaction étant catalysée par du palladium (0), pour conduire à l'aldéhyde correspondant de formule (XXI) : 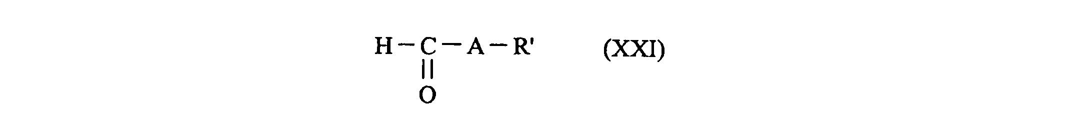
dans laquelle A et R' sont tels que définis précédemment, composé de formule (XXI) pouvant par ailleurs être obtenu par des techniques classiques de lithiations à partir du dérivé halogéné de formule (XX), ou par l'intermédiaire du dérivé vinylique correspondant (obtenu à partir du composé de formule (XX) par action de vinyltributylétain et de palladium tetrakis) soumis à une ozonolyse, ou encore par formylation directe du noyau A selon une réaction de Vilsmeier par exemple,
composé de formule (XXI) que l'on soumet à un agent oxydant pour obtenir le composé de formule (XXII) :
HOOC―A-R' (XXII)
dans laquelle A et R' sont définis comme précédemment, qui est transformé, après l'action de chlorure de thionyle et d'un azidure, puis d'un acide, en composé de formule (I/g), cas particulier des composés de formule (I) :
H₂N―A―R' (I/g)
dans laquelle A et R' sont définis comme précédemment, sur lequel on condense une ou deux molécules d'un composé de formule (XVIII) :
R₁ₐ―J (XVIII)
dans laquelle R₁ₐ peut prendre toutes les valeurs du groupement Rₐ tel que défini précédemment à l'exception de l'atome d'hydrogène et J représente un groupe partant comme un atome d'halogène ou un groupement tosyle,
pour obtenir le composé de formule (I/h), cas particulier des composés de formule (I) :
R'₂ₐR₂ₐN―A―R' (I/h)
dans laquelle A et R' ont la même signification que précédemment et R'₂ₐ et R₂ₐ, identiques ou différents, représentent un groupement Rₐ avec la condition suivante : R'₂ₐ et R₂ₐ ne peuvent représenter simultanément un atome d'hydrogène et ne peuvent former, avec l'atome d'azote qui les porte un groupement cyclique,
ou composé de formule (XX) qui est soumis, dans des conditions de substitution nucléophile aromatique, à l'action d'une amine R'ₐR''ₐNH dans laquelle R'ₐ et R''ₐ sont tels que définis précédemment (R'ₐ et R''ₐ peuvent entre autres former avec l'atome d'azote qui les porte un groupement cyclique tel que défini précédemment), pour conduire au composé de formule (I/i), cas particulier des composés de formule (I) :
R'ₐR"ₐN―A―R' (I/i)
dans laquelle R'ₐ, R"ₐ, A et R' sont tels que définis précédemment,
les composés (I/g) à (I/i) pouvant être purifiés selon une technique classique de séparation, que l'on transforme, si on le souhaite, en leurs sels d'addition à un acide ou à une base pharmaceutiquement acceptable, et dont on sépare éventuellement les isomères selon une technique classique de séparation.

17. Composés de formule (XX_{A}) selon la revendication 16, cas particulier des composés de formule (XX) :
Hal―A_{A}―R'_{A} (XXA)
dans laquelle :
◆ Hal représente un atome d'halogène (fluor, chlore, brome, iode)
◆ A_{A} représente :
- un cycle de formule (a) : 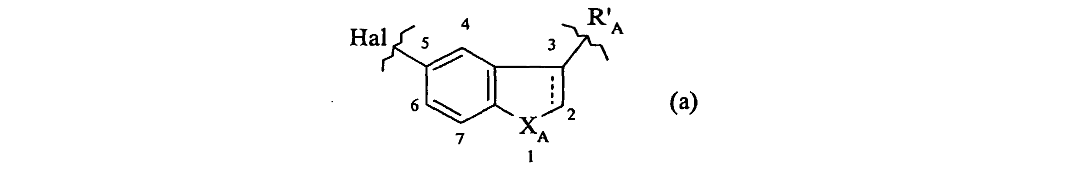 dans laquelle X_{A} représente un atome de soufre, ou un groupement NR₀ (dans lequel R₀ est tel que défini précédemment), et la représentation ...., est telle que définie dans la revendication 1,
- ou un cycle de formule (b) : 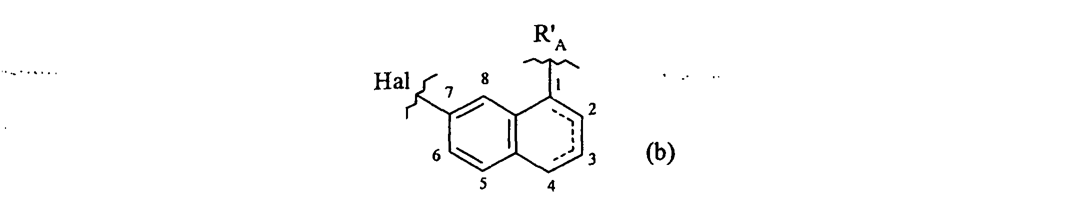 dans laquelle la représentation .... a la même signification que précédemment, les cycles de formule (a) ou (b) pouvant être substitués (en plus de l'atome d'halogène et du groupement R'_{A}) position 2 (formule (a)) ou 3 (formule (b)) par un groupement choisi parmi Rₐ, CORₐ, COORₐ, OCORₐ où Rₐ est tel que défini dans la revendication 16,
◆ et R'_{A} représente un groupement G-R²_{A} dans lequel G est tel que défini dans la revendication 1 et R²_{A} représente un groupement 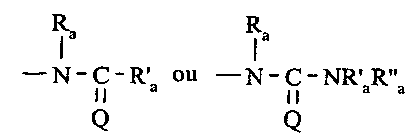 où Rₐ, R'ₐ, R"ₐ et Q sont tels que définis précédemment,
leurs énantiomères et diastéréoisomères, ainsi que leurs sels d'addition à un acide ou une base pharmaceutiquement acceptable,
en tant qu'intermédiaires de synthèse mais également en tant que composés utiles pour le traitement des troubles liés au système mélatoninergique.

18. Compositions pharmaceutiques contenant les produits de formule (I) selon l'une quelconque des revendications 1 à 15 et 17 ou un de leurs sels d'addition avec un acide ou une base pharmaceutiquement acceptable en combinaison avec un ou plusieurs excipients pharmaceutiquement acceptables.

19. Compositions pharmaceutiques selon la revendication 18 utiles pour la fabrication de médicaments pour le traitement des troubles liés au système mélatoninergique.

## Patentansprüche

1. Verbindungen der Formel (I):
R-A-R' (I)
in der:
◆ A:
- ein cyclisches System der Formel (II) bedeutet: 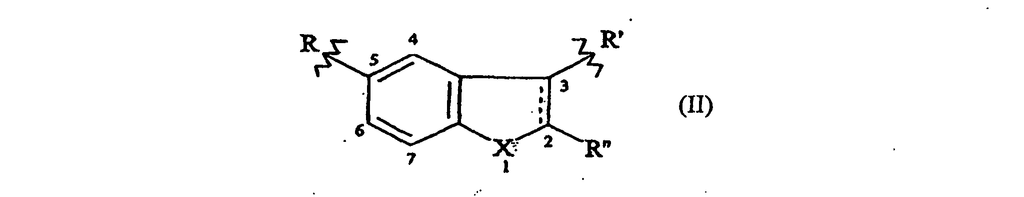 in der
● X ein Sauerstoff- oder Schwefelatom oder eine Gruppe NR₀ bedeutet (worin R₀ ein Wasserstoffatom, eine geradkettige oder verzweigte (C₁-C₆)-Alkylgruppe. Arylgruppe, geradkettige oder verzweigte Aryl-(C₁-C₆)-alkylgruppe oder Gruppe SO₂Ph darstellt),
● das Symbol ---- bedeutet, daß die Bindung einfach oder doppelt sein kann, mit der Maßgabe, daß die Wertigkeit der Atome berücksichtigt ist,
● R" eine Gruppe Rₐ, ORₐ, CORₐ, COORₐ, OCORₐ, OSO₂CF₃, Cyano, Nitro oder Halogenatome bedeutet,
worin Rₐ ein Wasserstoffatom, eine geradkettige oder verzweigte, substituierte oder unsubstituierte (C₁-C₆)-Alkylgruppe, geradkettige oder verzweigte, substituierte oder unsubstituierte (C₂-C₆)-Alkenylgruppe, geradkettige oder verzweigte, substituierte oder unsubstituierte (C₂-C₆)-Alkinylgruppe, geradkettige oder verzweigte (C₁-C₆)-Polyhalogenalkylgruppe, substituierte oder unsubstituierte (C₃-C₈)-Cycloalkylgruppe, geradkettige oder verzweigte, substituierte oder unsubstituierte (C₃-C₈)-Cycloalkyl-(C₁-C₆)-alkylgruppe, substituierte oder unsubstituierte (C₃-C₈)-Cycloalkenylgruppe, geradkettige oder verzweigte, substituierte oder unsubstituierte (C₃-C₈)-Cycloalkenyl-(C₁-C₆)-alkylgruppe, Arylgruppe, geradkettige oder verzweigte Aryl-(C₁-C₆)-alkylgruppe, geradkettige oder verzweigte Aryl-(C₁-C₆)-alkenylgruppe, Heteroarylgruppe, geradkettige oder verzweigte Heteroaryl-(C₁-C₆)-alkylgruppe, geradkettige oder verzweigte Heteroaryl - (C₁-C₆) - alkenylgruppe, substituierte oder unsubstituierte Heterocycloalkylgruppe, substituierte oder unsubstituierte Heterocycloalkenylgruppe, geradkettige oder verzweigte, substituierte oder unsubstituierte Heterocycloalkyl-(C₁-C₆)-alkylgruppe oder geradkettige oder verzweigte, substituierte oder unsubstituierte Heterocycloalkenyl-(C₁-C₆)-alkylgruppe darstellt,
- oder ein cyclisches System der Formel (III) bedeutet: 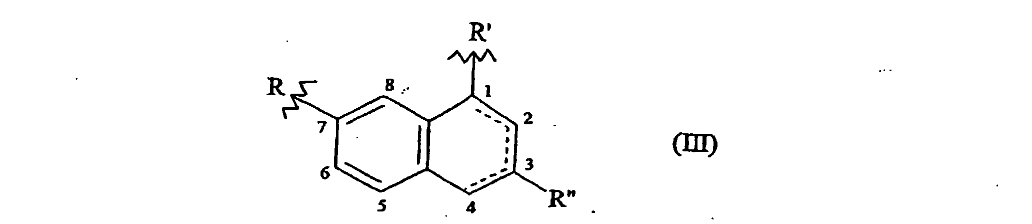 worin R" und das Symbol ---- die oben angegebenen Bedeutungen besitzen,
◆ R: eine Gruppe -NR'ₐR''ₐ bedeutet, worin R'ₐ und R''ₐ, die gleich- oder verschiedenartig sind, sämtliche Werte von Rₐ annehmen können und auch zusammen mit dem sie tragenden Stickstoffatom eine cyclische Gruppe bilden können, die 5 bis 10 Kettenglieder aufweisen kann und zusätzlich zu dem Stickstoffatom ein bis drei Heteroatome ausgewählt aus Sauerstoff, Schwefel und Stickstoff enthalten kann,
◆ und R' eine Gruppe der Formel (VII) bedeutet:
-G-R² (VII)
in der
● G eine Alkylenkette -(CH₂)ₜ- darstellt (worin t den Wert 2 oder 3 besitzt),
● und R² eine Gruppe 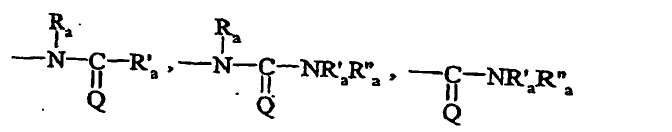 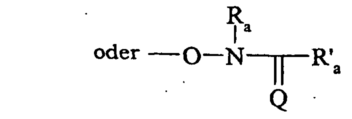 darstellt, worin Q ein Sauerstoff- oder Schwefelatom darstellt, und Rₐ, R'ₐ und R"ₐ (die gleich oder verschiedenartig sind) die oben angegebenen Bedeutungen besitzen, wobei R'ₐ und R"ₐ zusammen mit dem sie tragenden Stickstoffatom eine cyclische Gruppe, wie sie oben definiert worden ist, bilden können, mit der Maßgabe, daß:
- man unter "Heterocycloalkylgruppe" jede gesättigte, mono- oder polycyclische Gruppe, die 5 bis 10 Atome enthält, die 1 bis 3 Heteroatome ausgewählt aus Stickstoff, Sauerstoff oder Schwefel umfassen, versteht,
- man unter "Heterocycloalkenylgruppe" jede mono- oder polycyclische nichtaromatische Gruppe, die eine oder mehrere Unsättigungen aufweist, 5 bis 10 Atome enthält, die 1 bis 3 Heteroatome ausgewählt aus Stickstoff, Sauerstoff und Schwefel umfassen können, versteht,
- der Begriff "substituiert" unter Bezug auf die Begriffe "Alkyl", "Alkenyl" und "Alkinyl" bedeutet, daß diese Gruppen durch eine oder mehrere gleiche oder verschiedenartige Reste ausgewählt aus Hydroxy, geradkettigem oder verzweigtem (C₁-C₆)-Alkoxy, geradkettigem oder verzweigtem (C₁-C₆)-Alkyl, geradkettigem oder verzweigtem (C₁-C₆)-Polyhalogenalkyl, Amino oder Halogenatomen substituiert sind,
- der Begriff "substituiert" unter Bezug auf die Begriffe "Cycloalkyl", "Cycloalkylalkyl", "Cycloalkenyl", "Cycloalkenylalkyl", "Heterocycloalkyl", "Heterocycloalkenyl", "Heterocycloalkylalkyl" und "Heterocycloalkenylalkyl" bedeutet, daß der cyclische Rest dieser Gruppen durch eine oder mehrere gleiche oder verschiedenartige Reste ausgewählt aus Hydroxy, geradkettigem oder verzweigtem (C₁-C₆)-Alkoxy, geradkettigem oder verzweigtem (C₁-C₆)-Alkyl, geradkettigem oder verzweigtem (C₁-C₆)-Polyhalogenalkyl, Amino oder Halogenatomen, substituiert ist,
- man unter "Aryl" jede mono- oder polycyclische aromatische Gruppe, die 6 bis 22 Kohlenstoffatome aufweist, einschließlich der Biphenylgruppe, versteht,
- man unter "Heteroaryl" jede aromatische, mono- oder polycyclische Gruppe, die 5 bis 10 Atome enthält, die 1 bis 3 Heteroatome ausgewählt aus Stickstoff, Sauerstoff oder Schwefel umfassen, versteht,
wobei die "Aryl"- und "Heteroaryl"-gruppen durch eine oder mehrere, gleiche oder verschiedenartige Reste ausgewählt aus Hydroxy, geradkettigem oder verzweigtem (C₁-C₆)-Alkoxy, geradkettigein oder verzweigtem (C₁-C₆)-Alkyl, geradkettigem oder verzweigtem (C₁-C₆)-Polyhalogenalkyl, Cyano, Nitro, Amino oder Halogenatomen substituiert sein können,
mit der Maßgabe, daß:
- wenn A einen in der 2-Stellung durch eine gegebenenfalls substituierte Phenylgruppe substituierten Indolkern bedeutet, dann R² keine Gruppe
- NHCORₑ bedeuten kann, in der Rₑ eine Gruppe darstellt, die einen mono- oder bicyclischen, aromatischen oder nichtaromatischen Heterocyclus enthält,
deren Enantiomere und Diastereoisomere sowie deren Additionssalze mit einer pharmazeutisch annehmbaren Säure oder Base.

2. Verbindungen der Formel (I) nach Anspruch 1, worin R' eine Gruppe G-R² bedeutet, in der G eine Alkylenkette -(CH₂)ₜ- darstellt, worin t 2 oder 3 bedeutet, und R² eine Gruppe -NHCOR'ₐ oder -CONHR'ₐ darstellt, worin R'ₐ die in Anspruch 1 angegebenen Bedeutungen besitzt, deren Enantiomere und Diastereoisomere sowie deren Additionssalze mit einer pharmazeutisch annehmbaren Säure oder Base.

3. Verbindungen der Formel (I) nach Anspruch 1, worin A ein cyclisches System der Formel (II) bedeutet, deren Enantiomere und Diastereoisomere sowie deren Additionssalze mit einer pharmazeutisch annehmbaren Säure oder Base.

4. Verbindungen der Formel (I) nach Anspruch 1, worin A ein cyclisches System der Formel (III) bedeutet, deren Enantiomere und Diastereoisomere sowie deren Additionssalze mit einer pharmazeutisch annehmbaren Säure oder Base.

5. Verbindungen der Formel (I) nach Anspruch 1, worin A ein cyclisches System der Formel (II) bedeutet und R' eine Gruppe der Formel (VII) darstellt, in der R² eine Gruppe 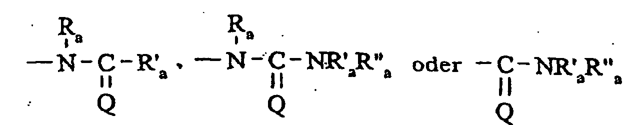 darstellt,
worin Q, Rₐ, R'ₐ und R"ₐ die in Anspruch 1 angegebenen Bedeutungen besitzen, deren Enahtiomere und Diastereoisomere sowie deren Additionssalze mit einer pharmazeutisch annehmbaren Säure oder Base.

6. Verbindungen der Formel (I) nach Anspruch 1, worin A ein cyclisches System der Formel (III) bedeutet und R' eine Gruppe der Formel (VII) darstellt, in der R² eine Gruppe 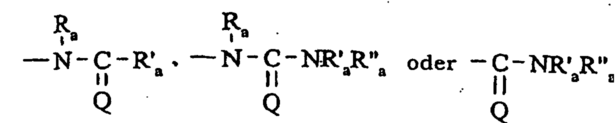 darstellt,
worin Q, Rₐ, R'ₐ und R"ₐ die in Anspruch 1 angegebenen Bedeutungen besitzen, deren Enantiomere und Diastereoisomere sowie deren Additionssalze mit einer pharmazeutisch annehmbaren Säure oder Base.

7. Verbindungen der Formel (I) nach Anspruch 1, worin A einen Naphthalin-, Dihydronaphthalin- oder Tetrahydronaphthalinkern bedeutet, deren Enantiomere und Diastereoisomere sowie deren Additionssalze mit einer pharmazeutisch annehmbaren Säure oder Base.

8. Verbindungen der Formel (I) nach Anspruch 1, worin A einen Benzofuran- oder Dihydrobenzofurankern bedeutet, deren Enantiomere und Diastereoisomere sowie deren Additionssalze mit einer pharmazeutisch annehmbaren Säure oder Base.

9. Verbindungen der Formel (I) nach Anspruch 1, worin A einen Benzothiophen- oder Dihydrobenzothiophenkern bedeutet, deren Enantiomere und Diastereoisomere sowie deren Additionssalze mit einer pharmazeutisch annehmbaren Säure oder Base.

10. Verbindungen der Formel (I) nach Anspruch 1, worin A einen Indol- oder Indolinkern bedeutet, deren Enantiomere und Diastereoisomere sowie deren Additionssalze mit einer pharmazeutisch annehmbaren Säure oder Base.

11. Verbindung der Formel (I) nach Anspruch 1, worin A einen Naphthalin-, Dihydronaphthalin- oder Tetrahydronaphthalinkern bedeutet und R' eine Gruppe -(CH₂)ₜ-NHCOR'ₐ oder -(CH₂)ₜ-CONHR'ₐ darstellt, worin t den Wert 2 oder 3 besitzt und R'ₐ die in Anspruch 1 angegebenen Bedeutungen besitzt, deren Enantiomere und Diastereoisomere sowie deren Additionssalze mit einer pharmazeutisch annehmbaren Säure oder Base.

12. Verbindungen der Formel (I) nach Anspruch 1, worin A einen Benzofuran- oder Dihydrobenzofurankern bedeutet und R' eine Gruppe -(CH₂)ₜ-NHCOR'ₐ oder -(CH₂)ₜ-CONHR'ₐ darstellt, worin t den Wert 2 oder 3 besitzt und R'ₐ die in Anspruch 1 angegebenen Bedeutungen besitzt, deren Enantiomere und Diastereoisomere sowie deren Additionssalze mit einer pharmazeutisch annehmbaren Säure oder Base.

13. Verbindungen der Formel (I) nach Anspruch 1, worin A einen Benzothiophen- oder Dihydrobenzothiophenkern bedeutet und R' eine Gruppe -(CH₂)ₜ-NHCOR'ₐ oder -(CH₂)ₜ-CONHR'ₐ darstellt, worin t den Wert 2 oder 3 besitzt und R'ₐ die in Anspruch 1 angegebenen Bedeutungen besitzt, deren Enantiomere und Diastereoisomere sowie deren Additionssalze mit einer pharmazeutisch annehmbaren Säure oder Base.

14. Verbindungen der Formel (I) nach Anspruch 1, worin A einen Indol- oder Indolinkern bedeutet und R' eine Gruppe -(CH₂)ₜ-NHCOR'ₐ oder -(CH₂)ₜ-CONHR'ₐ darstellt, worin t den Wert 2 oder 3 besitzt und R'ₐ die in Anspruch 1 angegebenen Bedeutungen aufweist, deren Enantiomere und Diastereoisomere sowie deren Additionssalze mit einer pharmazeutisch annehmbaren Säure oder Base.

15. Verbindungen der Formel (I) nach Anspruch 1, nämlich:
* N-{2-[7-Amino-3-(cyclopropylmethyl)-1-naphthyl]-ethyl}-acetamid,
* N-{2-[7-(Diethylamino)-1-naphthyl]-ethyl}-2-phenylacetamid,
* N-{2-[7-(Hexylamino)-1,2,3,4-tetrahydro-1-naphthalinyl]-ethyl}-acetamid,
* N-[(6-Morpholino-2-phenyl-*2H*-3-chromenyl]-methyl}-acetamid,
deren Enantiomere und Diastereoisomere sowie deren Additionssalze mit einer pharmazeutisch annehmbaren Säure oder Base.

16. Verfahren zur Herstellung der Verbindungen der Formel (I) nach Anspruch 1, **dadurch gekennzeichnet, daß** man als Ausgangsprodukt die Verbindung der Formel (VIII) verwendet: 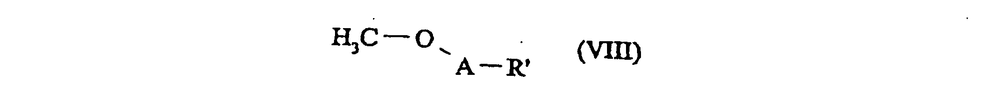 in der A und R' die oben angegebenen Bedeutungen besitzen, welche man einer Demethylierung unter Verwendung von klassischen Reagenzien, wie HBr, AlCl₃, AlBr₃, BBr₃ oder binären Lewis-Säuren/Nucleophil-Systemen, wie beispielsweise AlCl₃/PhCH₂SH oder BBr₃/Me₂S unterwirft, zur Bildung der Verbindung der Formel (IX):
HO-A-R' (IX)
in der A und R' die oben angegebenen Bedeutungen besitzen,
welche man durch die Einwirkung von Reagenzien, wie POCl₃, PCl₅, Ph₃PBr₂, PhPCl₄, HBr oder HI, in das entsprechende Halogenderivat der Formel (XX) umwandelt:
Hal―A-R' (XX)
in der A und R' die oben angegebenen Bedeutungen besitzen und Hal ein Halogenatom bedeutet (wobei die Verbindungen der Formel (XX) auch durch Austauschreaktionen erhalten werden, beispielsweise die Behandlung eines Chlorderivats mit KF in Dimethylformamid zur Bildung des entsprechenden Fluorderivats, oder durch Behandeln eines Bromderivats mit KI in Gegenwart von Kupfersalzen zur Bildung des entsprechenden Iodderivats, wobei die Verbindungen der Formel (XX) andererseits auch ausgehend von Verbindungen der Formel (XX₁) oder (XX₂) erhalten werden können: 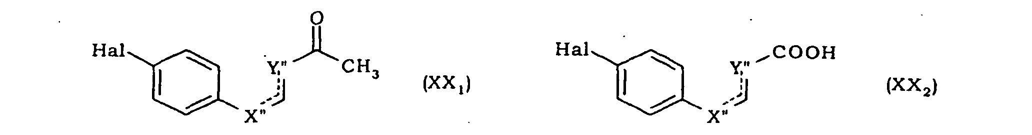 in der Hal die oben angegebenen Bedeutungen besitzen, X" ein Sauerstoff- oder Schwefelatom, eine Gruppe C(H)_{q} (worin q 0, 1 oder 2 ist), oder eine Gruppe NR₀ (worin R₀ die oben angegebenen Bedeutung besitzt), und Y" ein Bindung oder eine Gruppe C(H)_{q} (worin q 0, 1 oder 2 ist) bedeutet), welche Verbindung der Formel (XX) : entweder mit Kohlenmonoxid und Bu₃SnH behandelt wird, wobei die Reaktion durch Palladium (0) katalysiert wird, zur Bildung des entsprechenden Aldehyds der Formel (XXI) : 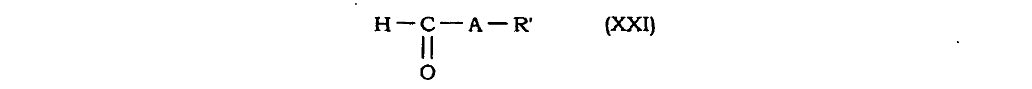 in der A und R' die oben angegebenen Bedeutungen besitzen, welche Verbindung der Formel (XXI) andererseits auch mit Hilfe klassischer Lithierungsmethoden ausgehend von dem Halogenderivat der Formel (XX) oder über das entsprechende Vinylderivat (welches ausgehend von der Verbindung der Formel (XX) durch Einwirkung von Vinyltributylzinn und Palladiumtetrakis erhalten wird, welches einer Ozonolyse unterworfen wird; oder durch direkte Formylierung des Kerns A gemäß beispielsweise einer Vilsmeier-Reaktion erhalten werden kann, welche Verbindung der Formel (XXI) man der Einwirkung eines Oxidationsmittels unterwirft zur Bildung der Verbindung der Formel (XXII) :
HOOC―A-R' (XXII)
in der A und R' die oben angegebenen Bedeutungen besitzen, welche man nach der Umsetzung mit Thionylchlorid und einem Azid und dann mit einer Säure in die Verbindung der Formel (I/g) umwandelt, einem Sonderfall der Verbindungen der Formel (I) :
H₂N―A―R' (I/g)
in der A und R' die oben angegebenen Bedeutungen besitzen, welche man mit einem oder zwei Molekülen einer Verbindung der Formel (XVIII) :
R₁ₐ―J (XVIII)
in der R₁ₐ die Rₐ oben angegebenen Bedeutung besitzt mit der Ausnahme des Hydrogen Atom, und J ein Rest Gruppe bedeutet so wie ein Hydrogen Atom oder ein Tosyl Gruppe,kondensiert zur Bildung der Verbindung der Formel (I/h), einem Sonderfall der Verbindungen der Formel (I) :
R'₂ₐR₂ₐN―A―R' (I/h)
in der A und R' die oben angegebenen Bedeutungen besitzen und R'₂ₐ und R₂ₐ, die gleich oder verschiedenartig sind, eine Gruppe Rₐ bedeuten, mit der Maßgabe, daß : R'₂ₐ und R₂ₐ nicht gleichzeitig ein Wasserstoffatom bedeuten können und mit dem sie tragenden Stickstoffatom nicht eine cyclische Gruppe bilden können, oder welche Verbindung der Formel (XX) man unter den Bedingungen der aromatischen nucleophilen Substitution der Einwirkung eines Amins R'ₐR"ₐNH, in der R'ₐ und R"ₐ die oben angegebenen Bedeutungen besitzen (wobei R'ₐ und R"ₐ unter anderem mit dem sie tragenden Stickstoffatom eine cyclische Gruppe bilden können, wie sie oben definiert worden ist) unterwirft zur Bildung der Verbindung der Formel (I/i), einem Sonderfall der Verbindungen der Formel (I):
R'ₐR"ₐN - A - R' (I/i)
in der R'ₐ, R"ₐ A und R' die oben angegebenen Bedeutungen besitzen,
welche Verbindungen (I/g) bis (I/i) mit Hilfe einer klassischen Trennmethode gereinigt werden können, welche man gewünschtenfalls in ihre Additionssalze mit einer pharmazeutisch annehmbaren Säure oder Base überführt und welche man gegebenenfalls mit Hilfe einer klassischen Trennmethode in ihre Isomeren auftrennt.

17. Verbindungen der Formel (XX_{A}) nach Anspruch 16, einem Sonderfall der Verbindungen der Formel (XX):
Hal - A_{A} - R'_{A} (XXA)
in der:
◆ Hal ein Halogenatom (Fluor, Chlor, Brom oder Iod) und
◆ A_{A}:
- einen Ring der Formel (a): 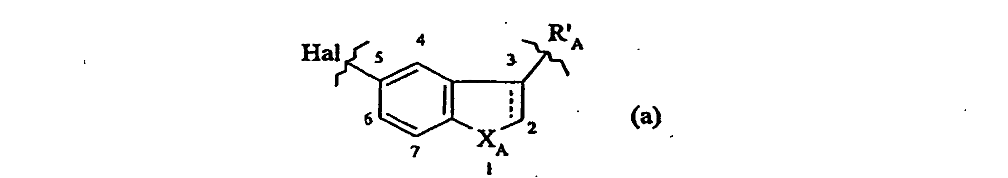 in der X_{A} ein Schwefelatom oder eine Gruppe NR₀ darstellt (worin R₀ die oben angegebenen Bedeutungen besitzt) und das Symbol ----- die in Anspruch 1 angegebenen Bedeutungen besitzt,
- oder einen Ring der Formel (b) bedeutet: 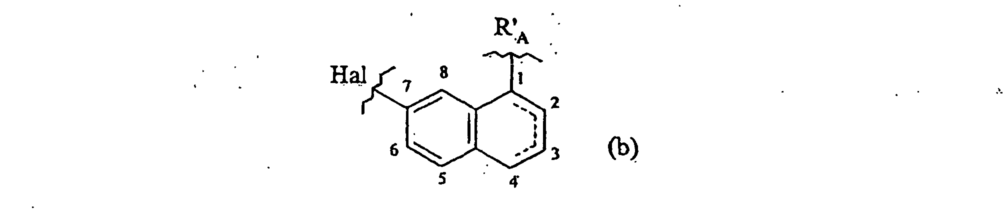 in der das Symbol ---- die oben angegebenen Bedeutungen besitzt,
wobei die Ringe der Formeln (a) oder (b) (neben dem Halogenatom und der Gruppe R'_{A}) in der Position 2 (Formel a)) oder 3 (Formel b)) durch eine Gruppe ausgewählt aus Rₐ, CORₐ, COORₐ, OCORₐ, worin Rₐ die in Anspruch 16 angegebenen Bedeutungen besitzt, substituiert sein können,
◆ und R'_{A} eine Gruppe G-R²_{A} darstellt, worin G die in Anspruch 1 angegebenen Bedeutungen besitzt und R²_{A} eine Gruppe 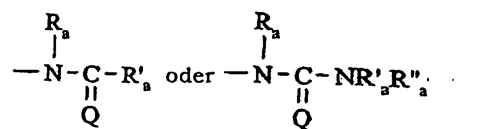 darstellt, worin Rₐ, R'ₐ, R"ₐ und Q die oben angegebenen Bedeutungen besitzen,
deren Enantiomere und Diastereoisomere sowie deren Additionssalze mit einer pharmazeutisch annehmbaren Säure oder Base,
als Synthese-Zwischenprodukte als auch als Verbindungen, die zur Behandlung von Störungen nützlich sind, die mit dem melatoninergischen System verknüpft sind.

18. Pharmazeutische Zubereitungen enthaltend die Produkte der Formel (I) nach einem der Ansprüche 1 bis 15 und 17 oder eines ihrer Additionssalze mit einer pharmazeutisch annehmbaren Säure oder Base in Kombination mit einem oder mehreren pharmazeutisch annehmbaren Hilfsstoffen.

19. Pharmazeutische Zubereitungen nach Anspruch 18 für die Herstellung von Arzneimitteln zur Behandlung von Störungen, die mit dem melatoninergischen System verknüpft sind.

## Claims

1. Compounds of formula (I) :
R-A-R' (I)
wherein:
◆ A represents :
― a ring system of formula (II) : 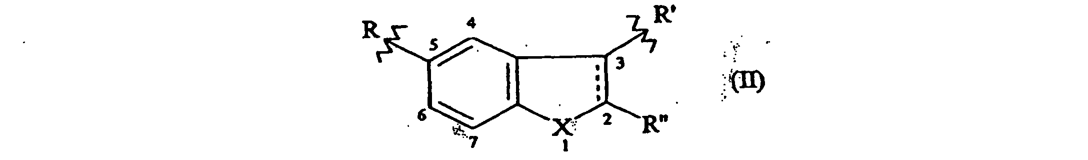 wherein
• X represents an oxygen or sulphur atom or a group NR₀ (wherein R₀ represents a hydrogen atom, a linear or branched (C₁-C₆)alkyl group, an aryl group, an aryl-(C₁-C₆)alkyl group in which the alkyl moiety is linear or branched, or SO₂Ph),
• the symbol .... means that the bond may be single or double, it being understood that the valency of the atoms is respected,
• R" represents a Rₐ, ORₐ, CORₐ, COORₐ, OCORₐ, OSO₂CF₃, cyano or nitro group or halogen atoms,
wherein Rₐ represents a hydrogen atom, an unsubstituted or substituted linear or branched (C₁-C₆)alkyl group, an unsubstituted or substituted linear or branched (C₂-C₆)alkenyl group, an unsubstituted or substituted linear or branched (C₂-C₆)alkynyl group, a linear or branched (C₁-C₆)polyhaloalkyl group, an unsubstituted or substituted (C₃-C₈)cycloalkyl group, an unsubstituted or substituted (C₃-C₈)cycloalkyl-(C₁-C₆)alkyl group in which the alkyl moiety is linear or branched, an unsubstituted or substituted (C₃-C₈)cycloalkenyl group, an unsubstituted or substituted (C₃-C₈)cycloalkenyl-(C₁-C₆)alkyl group in which the alkyl moiety is linear or branched, an aryl group, an aryl-(C₁-C₆)alkyl group in which the alkyl moiety is linear or branched, an aryl-(C₁-C₆)alkenyl group in which the alkenyl moiety is linear or branched, a heteroaryl group, a heteroaryl-(C₁-C₆)alkyl group in which the alkyl moiety is linear or branched, a heteroaryl-(C₁-C₆)alkenyl group in which the alkenyl moiety is linear or branched, an unsubstituted or substituted heterocycloalkyl group, an unsubstituted or substituted heterocycloalkenyl group, an unsubstituted or substituted heterocycloalkyl-(C₁-C₆)alkyl group in which the alkyl moiety is linear or branched, or an unsubstituted or substituted heterocycloalkenyl-(C₁-C₆)alkyl group in which the alkyl moiety is linear or branched,
― or a ring system of formula (III) : 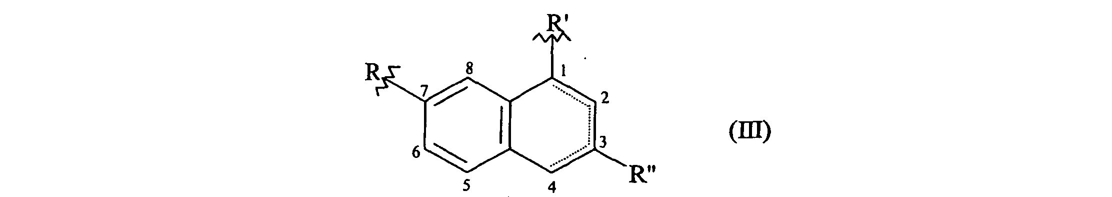 wherein R" and the symbol .... are as defined hereinbefore,
◆ R represents :
a group -NR'ₐR"ₐ wherein R'ₐ and R"ₐ, which may be the same or different, may take any of the values of Rₐ and also may form, together with the nitrogen atom carrying them, a 5- to 10-membered cyclic group which may contain, in addition to the nitrogen atom, from one to three hetero atoms selected from oxygen, sulphur and nitrogen,
◆ and R' represents a group of formula (VII) :
―G―R² (VII)
wherein
• G represents an alkylene chain -(CH₂)ₜ- (wherein t is 2 or 3),
• and R² represents a group 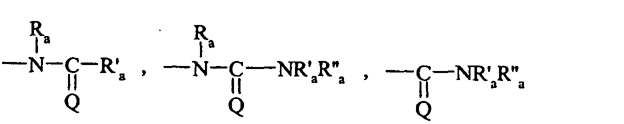 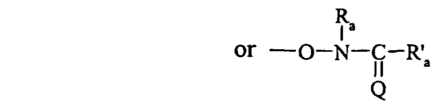 wherein Q represents an oxygen or sulphur atom, and Rₐ, R'a and R"a (which may be the same or different) are as defined hereinbefore, it being possible for R'ₐ and R"ₐ to form, together with the nitrogen atom carrying them, a cyclic group as defined hereinbefore, it being understood that :
― "heterocycloalkyl" is taken to mean any saturated mono- or poly-cyclic group containing from 5 to 10 atoms including from 1 to 3 hetero atoms selected from nitrogen, oxygen and sulphur,
― "heterocycloalkenyl" is taken to mean any non-aromatic mono- or poly-cyclic group containing one or more unsaturated bonds, containing from 5 to 10 atoms and which may contain from 1 to 3 hetero atoms selected from nitrogen, oxygen and sulphur,
― the term "substituted" used in respect of the expressions "alkyl", "alkenyl" and "alkynyl" indicates that the groups in question are substituted by one or more radicals, which may be the same or different, selected from hydroxy, linear or branched (C₁-C₆)alkoxy, linear or branched (C₁-C₆)alkyl, linear or branched (C₁-C₆)polyhaloalkyl, amino and halogen atoms,
― the term "substituted" used in respect of the expressions "cycloalkyl", "cycloalkylalkyl", "cycloalkenyl", "cycloalkenylalkyl", "heterocycloalkyl", "heterocycloalkenyl", "heterocycloalkylalkyl" and "heterocycloalkenylalkyl" indicates that the cyclic moiety of the groups in question is substituted by one or more radicals, which may be the same or different, selected from hydroxy, linear or branched (C₁-C₆)alkoxy, linear or branched (C₁-C₆)alkyl, linear or branched (C₁-C₆)polyhaloalkyl, amino and halogen atoms,
― "aryl" is taken to mean any aromatic, mono- or poly-cyclic group containing from 6 to 22 carbon atoms, and also the biphenyl group,
― "heteroaryl" is taken to mean any aromatic mono- or poly-cyclic group containing from 5 to 10 atoms including from 1 to 3 hetero atoms selected from nitrogen, oxygen and sulphur, it being possible for the "aryl" and "heteroaryl" groups to be substituted by one or more radicals, which may be the same or different, selected from hydroxy, linear or branched (C₁-C₆)alkoxy, linear or branched (C₁-C₆)alkyl, linear or branched (C₁-C₆)polyhaloalkyl, cyano, nitro, amino and halogen atoms,
it being understood that:
― when A represents an indole nucleus substituted in the 2-position by an optionally substituted phenyl group, then R² cannot represent a group -NHCORₑ wherein Rₑ is a group containing an aromatic or non-aromatic mono- or bi-cyclic heterocycle,
their enantiomers and diastereoisomers, and addition salts thereof with a pharmaceutically acceptable acid or base.

2. Compounds of formula (I) according to claim 1, wherein R' represents a group G-R² wherein G represents an alkylene chain -(CH₂)ₜ-, wherein t is 2 or 3, and R² represents a group -NHCOR'ₐ or -CONHR'ₐ wherein R'ₐ is as defined in claim 1, their enantiomers and diastereoisomers, and addition salts thereof with a pharmaceutically acceptable acid or base.

3. Compounds of formula (I) according to claim 1, wherein A represents a ring system of formula (II), their enantiomers and diastereoisomers, and addition salts thereof with a pharmaceutically acceptable acid or base.

4. Compounds of formula (I) according to claim 1, wherein A represents a ring system of formula (III), their enantiomers and diastereoisomers, and addition salts thereof with a pharmaceutically acceptable acid or base.

5. Compounds of formula (I) according to claim 1, wherein A represents a ring system of formula (II) and R' represents a group of formula (VII) wherein R² represents a group 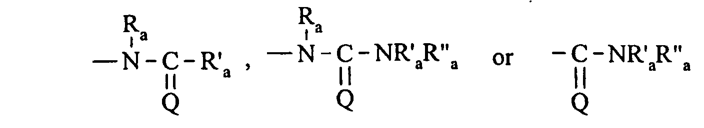 wherein Q, Rₐ, R'ₐ and R"ₐ are as defined in claim 1, their enantiomers and diastereoisomers, and addition salts thereof with a pharmaceutically acceptable acid or base.

6. Compounds of formula (I) according to claim 1, wherein A represents a ring system of formula (III) and R' represents a group of formula (VII) wherein R² represents a group 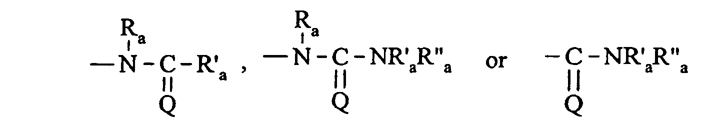 wherein Q, Rₐ, R'ₐ and R"ₐ are as defined in claim 1, their enantiomers and diastereoisomers, and addition salts thereof with a pharmaceutically acceptable acid or base.

7. Compounds of formula (I) according to claim 1, wherein A represents a naphthalene, dihydro- or tetrahydro-naphthalene nucleus, their enantiomers and diastereoisomers, and addition salts thereof with a pharmaceutically acceptable acid or base.

8. Compounds of formula (I) according to claim 1, wherein A represents a benzofuran or dihydrobenzofuran nucleus, their enantiomers and diastereoisomers, and addition salts thereof with a pharmaceutically acceptable acid or base.

9. Compounds of formula (I) according to claim 1, wherein A represents a benzothiophene or dihydrobenzothiophene nucleus, their enantiomers and diastereoisomers, and addition salts thereof with a pharmaceutically acceptable acid or base.

10. Compounds of formula (I) according to claim 1, wherein A represents an indole or indoline nucleus, their enantiomers and diastereoisomers, and addition salts thereof with a pharmaceutically acceptable acid or base.

11. Compounds of formula (I) according to claim 1, wherein A represents a naphthalene, dihydro- or tetrahydro-naphthalene nucleus, and R' represents a group -(CH₂)ₜ-NHCOR'ₐ or -(CH₂)ₜ-CONHR'ₐ wherein t is 2 or 3 and R'ₐ is as defined in claim 1, their enantiomers and diastereoisomers, and addition salts thereof with a pharmaceutically acceptable acid or base.

12. Compounds of formula (I) according to claim 1, wherein A represents a benzofuran or dihydrobenzofuran nucleus, and R' represents a group -(CH₂)ₜ-NHCOR'ₐ or -(CH₂)ₜ-CONHR'ₐ wherein t is 2 or 3 and R'ₐ is as defined in claim 1, their enantiomers and diastereoisomers, and addition salts thereof with a pharmaceutically acceptable acid or base.

13. Compounds of formula (I) according to claim 1, wherein A represents a benzothiophene or dihydrobenzothiophene nucleus, and R' represents a group -(CH₂)ₜ-NHCOR'ₐ or -(CH₂)ₜ-CONHR'ₐ wherein t is 2 or 3 and R'ₐ is as defined in claim 1, their enantiomers and diastereoisomers, and addition salts thereof with a pharmaceutically acceptable acid or base.

14. Compounds of formula (I) according to claim 1, wherein A represents an indole or indoline nucleus, and R' represents a group -(CH₂)ₜ-NHCOR'ₐ or -(CH₂)ₜ-CONHR'ₐ wherein t is 2 or 3 and R'ₐ is as defined in claim 1, their enantiomers and diastereoisomers, and addition salts thereof with a pharmaceutically acceptable acid or base.

15. Compounds of formula (I) according to claim 1 that are :
* N-{2-[7-amino-3-(cyclopropylmethyl)-1-naphthyl]ethyl}acetamide
* N-{2-[7-(diethylamino)-1-naphthyl]ethyl}-2-phenylacetamide
* N-{2-[7-(hexylamino)-1,2,3,4-tetrahydro-1-naphthyl]ethyl}acetamide
* N-[(6-morpholino-2-phenyl-*2H*-3-chromenyl)methyl]acetamide,
their enantiomers and diastereoisomers, and addition salts thereof with a pharmaceutically acceptable acid or base.

16. Process for the preparation of compounds of formula (I) according to claim 1, **characterised in that** there is used as starting material the compound of formula (VIII) : 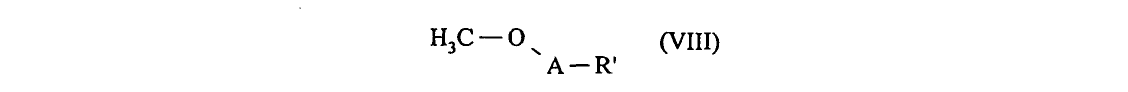 wherein A and R' are as defined hereinbefore, which is subjected to demethylation using conventional agents such as HBr, AlCl₃, AlBr₃, BBr₃ or Lewis acid/nucleophile binary systems such as AlCl₃/PhCH₂SH, or BBr₃/Me₂S, for example, to obtain the compound of formula (IX) :
HO-A―R' (IX)
wherein A and R' are as defined hereinbefore,
which is converted, by means of the action of reagents such as POCl₃, PCl₅, Ph₃PBr₂, PhPCl₄, HBr or HI, into the corresponding halogenated compound of formula (XX) :
Hal―A-R' (XX)
wherein A and R' are as defined hereinbefore and Hal represents a halogen atom (which compounds of formula (XX) can be obtained by exchange reactions such as, for example, the treatment of a chlorinated compound with KF in dimethylformamide to yield the corresponding fluorinated compound or the treatment of a brominated compound with KI in the presence of copper salts to yield the corresponding iodinated compound, and which compounds of formula (XX) can also be obtained starting from compounds of formula (XX₁) or (XX₂) : 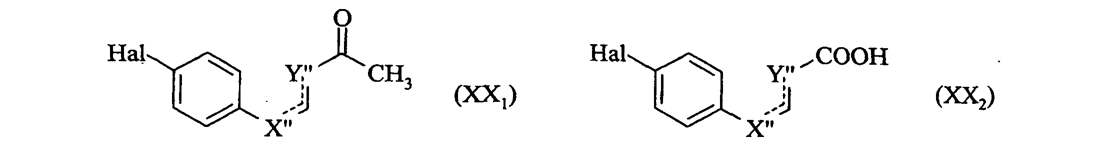 wherein Hal is as defined hereinbefore, X" represents an oxygen or a sulphur atom, a C(H)_{q} group (wherein q is 0, 1 or 2) or a NR₀ group (wherein R₀ is as defined hereinbefore), and Y" represents a bond or a C(H)_{q} group (wherein q is 0, 1 or 2)),
which compound of formula (XX) is :
• either treated with carbon monoxide and Bu₃SnH, the reaction being catalysed with palladium(0), to yield the corresponding aldehyde of formula (XXI) : 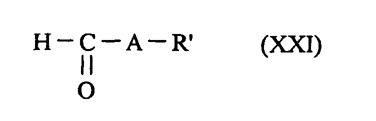
wherein A and R' are as defined hereinbefore,
which compound of formula (XXI) may alternatively be obtained by customary lithiation methods starting from the halogenated compound of formula (XX), or via the corresponding vinyl compound (obtained starting from the compound of formula (XX) by the action of vinyltributyltin and tetrakis palladium) subjected to ozonolysis, or furthermore by direct formylation of the nucleus A, for example according to a Vilsmeier reaction,
which compound of formula (XX) is subjected to an oxidising agent to obtain the compound of formula (XXII) :
HOOC―A―R' (XXII)
wherein A and R' are as defined hereinbefore, which is converted, by the action of thionyl chloride and an azide, and then on an acid, into the compound of formula (I/g), a particular case of the compounds of formula (I):
H₂N-A-R' (I/g)
wherein A and R' are as defined hereinbefore, with which there is condensed one or two molecules of a compound of formula (XVIII) :
R₁ₐ―J (XVIII)
wherein R₁ₐ may have all the meanings of the group Rₐ as defined hereinbefore except for the hydrogen atom and J represents a leaving group such as halogen atom or a tosyl group, to obtain the compound of formula (I/h), a particular case of the compounds of formula (I) :
R'₂ₐR₂ₐN―A―R' (I/h)
wherein A and R' are as defined hereinbefore and R'₂ₐ and R₂ₐ, which may be the same or different, represent a group Rₐ with the following proviso : R'₂ₐ and R₂ₐ cannot simultaneously represent a hydrogen atom and cannot form, together with the nitrogen atom carrying them, a cyclic group,
or which compound of formula (XX) is subjected, under conditions of nucleophilic aromatic substitution, to the action of an amine R'ₐR"ₐNH, wherein R'ₐ and R"ₐ are as defined hereinbefore (R'ₐ and R"ₐ may, *inter alia,* form, together with the nitrogen atom carrying them, a cyclic group as defined hereinbefore), to yield the compound of formula (I/j), a particular case of the compounds of formula (I) :
R'ₐR"ₐN―A―R' (I/i)
wherein R'ₐ, R"ₐ, A and R' are as defined hereinbefore,
which compounds (I/g) to (I/I) can be purified in accordance with a conventional separation technique, are converted, if desired, into their addition salts with a pharmaceutically acceptable acid or base and, optionally, are separated into their isomers in accordance with a conventional separation technique.

17. Compounds according to claim 16, of formula (XX_{A}), a particular case of the compounds of formula (XX) :
Hal―A_{A}―R'_{A} (XXA)
wherein:
◆ Hal represents a halogen atom (fluorine, chlorine, bromine, iodine),
◆ A_{A} represents:
- a ring system of formula (a) : 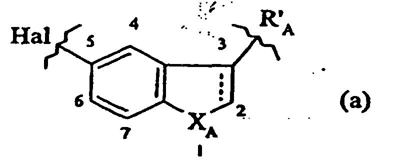 wherein X_{A} represents a sulphur atom or a group NR₀ (wherein R₀ is as defined hereinbefore), and the symbol .... is as defined in claim 1,
- or a ring system of formula (b) : 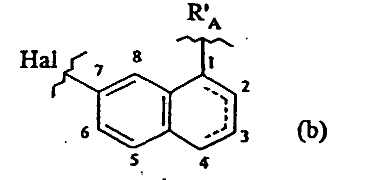 wherein the symbol .... is as defined hereinbefore,
which ring systems of formula (a) or (b) may be substituted (in addition to the halogen atom and the group R'_{A}) in the 2-position (formula (a)) or 3-position (formula (b)) by a group selected from Rₐ, CORₐ, COORₐ, OCORₐ wherein Rₐ is as defined in claim 16,
◆ and R'_{A} represents a group G-R²_{A} wherein G is as defined in claim 1 and R²_{A} represents 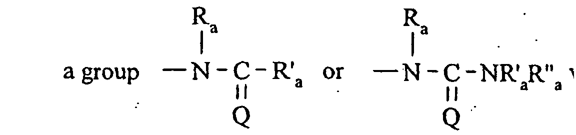 wherein Rₐ, R'ₐ, R"ₐ and Q are as defined hereinbefore,
their enantiomers and diastereoisomers, and addition salts thereof with a pharmaceutically acceptable acid or base,
as synthesis intermediates but also as compounds for use in the treatment of disorders associated with the melatoninergic system.

18. Pharmaceutical compositions comprising compounds of formula (I) according to any one of claims 1 to 15 and 17 or an addition salt thereof with a pharmaceutically acceptable acid or base, in combination with one or more pharmaceutically acceptable excipients.

19. Pharmaceutical compositions according to claim 18 for use in the manufacture of medicaments for the treatment of disorders associated with the melatoninergic system.
